# EUROPEAN PATENT SPECIFICATION

(11) **EP 3 496 530 B1**
(45) Date of publication and mention of the grant of the patent: **30.03.2022**
(21) Application number: 17840360.6
(22) Date of filing: 11.08.2017
(51) Int. Cl.: A01K 67/027, A61K 49/00, A61P 7/06, A61P 33/06

(54) **METHODS AND COMPOSITIONS RELATING TO IMPROVED HUMAN RED BLOOD CELL SURVIVAL IN GENETICALLY MODIFIED IMMUNODEFICIENT NON-HUMAN ANIMALS**
VERFAHREN UND ZUSAMMENSETZUNGEN IM ZUSAMMENHANG MIT DER VERBESSERTEN ÜBERLEBENSFÄHIGKEIT VON MENSCHLICHEN ROTEN BLUTZELLEN IN GENETISCH MODIFIZIERTEN IMMUNDEFIZIENTEN NICHTMENSCHLICHEN TIEREN
PROCÉDÉS ET COMPOSITIONS LIÉS À LA SURVIE AMÉLIORÉE DE GLOBULES ROUGES HUMAINS DANS DES ANIMAUX NON HUMAINS IMMUNODÉFICIENTS GÉNÉTIQUEMENT MODIFIÉS

(30) Priority: 11.08.2016 US 201662373671 P
(43) Date of publication of application: 19.06.2019
(60) Divisional application: 22157551.7
(73) Proprietor: The Jackson Laboratory, Bar Harbor, ME 04609 (US)
(72) Inventor: SHULTZ, Leonard D., Bar Harbor, ME 04609 (US); WILES, Michael V., Mount Desert, ME 04660 (US)
(74) Representative: Potter Clarkson
(86) International application number: PCT/US2017/046566
(87) International publication number: WO 2018/031920

(56) References cited:
- WO-A1-2009/114410
- US-A1- 2016 157 470
- US-A1- 2016 157 470
- TSUJI M ET AL: "Establishment of a SCID mouse model having circulating human red blood cells and a possible growth of Plasmodium falciparum in the mouse", VACCINE, ELSEVIER, AMSTERDAM, NL, vol. 13, no. 15, 1995, pages 1389-1392, XP004057446, ISSN: 0264-410X, DOI: 10.1016/0264-410X(95)00081-B
- Z. HU ET AL: "Macrophages prevent human red blood cell reconstitution in immunodeficient mice", BLOOD, vol. 118, no. 22, 24 November 2011 (2011-11-24), pages 5938-5946, XP55233818, US ISSN: 0006-4971, DOI: 10.1182/blood-2010-11-321414
- ASHLEY M VAUGHAN ET AL: "Development of humanized mouse models to study human malaria parasite infection", FUTURE MICROBIOLOGY, vol. 7, no. 5, May 2012 (2012-05), pages 657-665, XP55234288, GB ISSN: 1746-0913, DOI: 10.2217/fmb.12.27
- ITAMAR GOREN ET AL: "A Transgenic Mouse Model of Inducible Macrophage Depletion", AMERICAN JOURNAL OF PATHOLOGY., vol. 175, no. 1, July 2009 (2009-07), pages 132-147, XP55487727, US ISSN: 0002-9440, DOI: 10.2353/ajpath.2009.081002
- TIAN BIN ET AL: "An HSV-TK transgenic mouse model to evaluate elimination of fibroblasts for fibrosis therapy", AMERICAN JOURNAL OF PATHOLOGY; [10640], ELSEVIER INC, US, vol. 163, no. 2, August 2003 (2003-08), pages 789-801, XP002634896, ISSN: 0002-9440, DOI: 10.1016/S0002-9440(10)63706-6
- VALLEJO M. NEUTROPENIA AND PROGENITOR CELL MOBILIZATION IN AP 3-B1 MUTANT PEARL MICE, [Online] 2013, BIRMINGHAM, XP055462753 Retrieved from the Internet: <URL:https://search.proquest.com/openview/0 a045bfacfd5dc4b56b9b2b3e855498c/1 ?pq- origsite=gscholar&cbl=18750&diss=y> [retrieved on 2017-10-02]
- MOSIER ET AL.: 'Homozygous scid/scid;beige/beige mice have low levels of spontaneous or neonatal T cell -induced B cell generation' J.EXP.MED vol. 177, 1993, pages 191 - 194, XP055462763

## Description

### GOVERNMENT SPONSORSHIP

This invention was made with government support under Grant No. R24 ODO18259 awarded by the National Institutes of Health. The government has certain rights in the invention.

### REFERENCE TO RELATED APPLICATION

This application claims priority from U.S. Provisional Patent Application Serial No. 62/373,671, filed August 11, 2016.

### FIELD OF THE INVENTION

General aspects of this disclosure relate to methods and compositions for assessment of human red blood cells in an immunodeficient genetically modified animal. In specific aspects, methods and compositions for assessment of human red blood cells in an immunodeficient genetically modified mouse are provided.

### BACKGROUND OF THE INVENTION

Blood disorders such as malaria, sickle-cell anemia and aplastic anemia affect much of the world's population, especially those of African descent. Currently, 3.2 billion people live in areas that are at risk of malaria transmission while 3 million individuals have the sickle cell trait (CDC 2016). Advanced treatment of these and other blood disorders has been limited and there is a continuing need for non-human animal models, for assays of putative pharmaceutical therapeutics and other treatments.

US 2016/157470 A1 relates to humanized CD47 mice demonstrating a higher level of red blood cells after transplantation of human hematopoietic stem cells.

Tsuji et al, 1995. Vaccine, 13(15): 1389-1392 relates to immune-deficient mice transplanted with human red blood cells for growing *Plasmodium falciparum.*

Hu et al, 2011. Blood, 118(22): 5938-5946 relates to NSG mice that receive CD34+ cells and teaches that macrophages prevent human blood reconstitution in said mice.

Vaughan et al, 2012. Future Microbiol., 7(5): 657-665 reviews the use of NSG mice treated with liposomal-clodronate to deplete murine macrophages and co-administrated with human red blood cells and *Plasmodium falciparum* as a model of infection.

### SUMMARY OF THE INVENTION

The present invention is defined in the appended claims.

Otherwise described herein is a genetically modified immunodeficient non-human animal whose genome includes a genetic modification that renders the non-human animal deficient in macrophages and/or macrophage anti-human red blood cell activity so as to prolong the survival of human red blood cells when administered into said non-human animal.

In a first aspect, the present invention relates to a genetically modified immunodeficient mouse whose genome includes a genetic modification that renders the mouse deficient in macrophages and/or macrophage anti-human red blood cell activity so as to prolong the survival of human red blood cells when administered into said mouse, wherein the genetic modification is a mutation of a lysosomal trafficking regulator gene, wherein the mouse is a Lyst^{null} immunodeficient mouse.

Otherwise described herein is a genetically modified immunodeficient mouse whose genome includes a genetic modification that renders the mouse deficient in macrophages and/or macrophage anti-human red blood cell activity so as to prolong the survival of human red blood cells when administered into said mouse.

Otherwise described herein is a genetically modified NSG, NRG, or NOG mouse whose genome includes a genetic modification that renders the mouse deficient in macrophages and/or macrophage anti-human red blood cell activity so as to prolong the survival of human red blood cells when administered into said mouse.

Otherwise described herein is a genetically modified immunodeficient non-human animal whose genome includes a genetic modification that renders the non-human animal deficient in macrophages and/or macrophage anti-human red blood cell activity so as to prolong the survival of human red blood cells when administered into said non-human animal, wherein the genetic modification is a mutation of a lysosomal trafficking regulator (Lyst) gene such that the non-human animal does not express functional lysosomal trafficking regulator protein, rendering the non-human animal deficient in macrophages and/or macrophage anti-human red blood cell activity.

Otherwise described herein is a genetically modified immunodeficient mouse whose genome includes a genetic modification that renders the mouse deficient in macrophages and/or macrophage anti-human red blood cell activity so as to prolong the survival of human red blood cells when administered into said mouse, wherein the genetic modification is a mutation of a mouse lysosomal trafficking regulator gene such that the mouse does not express functional lysosomal trafficking regulator protein, rendering the mouse deficient in macrophages and/or macrophage anti-human red blood cell activity.

Otherwise described herein is a genetically modified immunodeficient mouse whose genome includes a genetic modification that renders the mouse deficient in macrophages and/or macrophage anti-human red blood cell activity so as to prolong the survival of human red blood cells when administered into said, wherein the genetic modification is a mutation of a mouse lysosomal trafficking regulator gene such that the mouse does not express functional lysosomal trafficking regulator protein, rendering the mouse deficient in macrophages and/or macrophage anti-human red blood cell activity and wherein the mutation comprises deletion of a 25 bp sequence: GAGCCGGTAGCTTTGGTTCAACGGA (SEQ ID NO: 1) from exon 5 of the Lyst gene in the genome of the genetically modified immunodeficient mouse.

Otherwise described herein is a genetically modified NSG, NRG, or NOG mouse whose genome includes a genetic modification that renders the genetically modified NSG, NRG, or NOG mouse deficient in macrophages and/or macrophage anti-human red blood cell activity so as to prolong the survival of human red blood cells when administered into said genetically modified NSG, NRG, or NOG , wherein the genetic modification is a mutation of a mouse lysosomal trafficking regulator (Lyst) gene such that the genetically modified NSG, NRG, or NOG does not express functional lysosomal trafficking regulator protein, rendering the genetically modified NSG, NRG, or NOG deficient in macrophages and/or macrophage anti-human red blood cell activity.

According to the first aspect of the present invention, the genetically modified immunodeficient mouse is a Lyst^{null} immunodeficient mouse.

According to the first aspect of the present invention, the genetically modified NSG, NRG, or NOG mouse is a Lyst^{null} NSG, NRG, or NOG mouse.

Otherwise described herein is an NSG, NRG, or NOG mouse that is homozygous for beige mutation Lyst^{bg}.

Otherwise described herein is a NOD.Cg-*Prkdc^{scid} Il2rg^{tm1Wjl}l*SzJ mouse homozygous for beige mutation Lyst^{bg}.

Otherwise described herein is a NOD.Cg*-Rag1^{tm1Mom} Il2rg^{tm1Wjl}l*SzJ mouse homozygous for beige mutation Lyst^{bg}.

Otherwise described herein is a NOD.Cg-*Prkdc^{scid} Il2rg^{tm1Sug}*/JicTac mouse homozygous for beige mutation Lyst^{bg}.

According to the first aspect of the present invention, the genetically modified immunodeficient mouse is a NOD.Cg-*Prkdc^{scid} Il2rg^{tm1Wjl}l*Lyst <emlMvw>/Sz (NSG Lyst knock out) mouse.

Otherwise described herein is a genetically modified immunodeficient non-human animal whose genome includes a genetic modification that renders the non-human animal deficient in macrophages and/or macrophage anti-human red blood cell activity so as to prolong the survival of human red blood cells when administered into said non-human animal wherein the genetic modification includes a transgene encoding human CD47 such that the non-human animal expresses human CD47 protein and further includes a mutation of its endogenous CD47 gene such that the endogenous CD47 is not expressed, rendering the genetically modified immunodeficient non-human animal deficient in macrophages and/or macrophage anti-human red blood cell activity.

Otherwise described herein is a genetically modified immunodeficient mouse whose genome includes a genetic modification that renders the mouse deficient in macrophages and/or macrophage anti-human red blood cell activity so as to prolong the survival of human red blood cells when administered into said genetically modified immunodeficient mouse wherein the genetic modification includes a transgene encoding human CD47 such that the mouse expresses human CD47 protein and further includes a mutation of mouse CD47 gene such that the mouse CD47 is not expressed, rendering the genetically modified immunodeficient mouse deficient in macrophages and/or macrophage anti-human red blood cell activity.

Otherwise described herein is a genetically modified NSG, NRG, or NOG mouse whose genome includes a genetic modification that renders the NSG, NRG, or NOG deficient in macrophages and/or macrophage anti-human red blood cell activity so as to prolong the survival of human red blood cells when administered into said genetically modified NSG, NRG, or NOG mouse wherein the genetic modification includes a transgene encoding human CD47 such that the genetically modified NSG, NRG, or NOG mouse expresses human CD47 protein and further includes a mutation of mouse CD47 gene such that the mouse CD47 is not expressed, rendering the genetically modified NSG, NRG, or NOG mouse deficient in macrophages and/or macrophage anti-human red blood cell activity.

Otherwise described herein is a genetically modified immunodeficient mouse is a NOD.Cg-Prkdc<scid> Cd47<tm1Fpl> Il2rg<tm1Wjl> Tg(CD47)2Sz/Sz (NSG Cd47 KO human CD47 Tg) mouse.

Otherwise described herein is a genetically modified immunodeficient non-human animal whose genome includes a genetic modification that renders the non-human animal deficient in macrophages and/or macrophage anti-human red blood cell activity so as to prolong the survival of human red blood cells when administered into said non-human animal wherein the genetic modification includes a transgene encoding herpes simplex virus 1 thymidine kinase such that the mouse expresses herpes simplex virus 1 thymidine kinase protein which, in combination with a nucleoside analog, renders the non-human animal deficient in macrophages.

Otherwise described herein is a genetically modified immunodeficient mouse whose genome includes a genetic modification that renders the genetically modified immunodeficient mouse deficient in macrophages and/or macrophage anti-human red blood cell activity so as to prolong the survival of human red blood cells when administered into said genetically modified immunodeficient mouse wherein the genetic modification includes a transgene encoding herpes simplex virus 1 thymidine kinase such that the genetically modified immunodeficient mouse expresses herpes simplex virus 1 thymidine kinase protein which, in combination with a nucleoside analog, renders the genetically modified immunodeficient mouse deficient in macrophages. A nucleoside analog such as ganciclovir, acyclovir or a combination thereof can be used.

Otherwise described herein is a genetically modified immunodeficient non-human animal further including human red blood cells administered into the blood system of the genetically modified immunodeficient non-human animal, such as by intraperitoneal (IP) or intravenous (IV) administration.

Otherwise described herein is a genetically modified immunodeficient mouse further including human red blood cells administered into the blood system of the genetically modified immunodeficient mouse, such as by intraperitoneal (IP) or intravenous (IV) administration.

Optionally, human hematopoietic cells (HSC) are administered to a genetically modified immunodeficient mouse of the present invention, such that human red blood cells are generated in the genetically modified immunodeficient mouse.

Human red blood cells survive longer in a genetically modified immunodeficient non-human animal, such as a genetically modified immunodeficient mouse, according to the first aspect of the present invention than in an immunodeficient mouse, of the same type whose genome does not include the genetic modification. For example, human red blood cells survive longer in a genetically modified NSG, NRG, or NOG mouse of the present invention than in an NSG, NRG, or NOG mouse whose genome does not include the genetic modification.

Optionally, the human red blood cells present in the genetically modified immunodeficient mouse according to the first aspect of the present invention, such as a genetically modified immunodeficient mouse of the said present invention, are infected by an infectious agent.

Optionally, an infectious agent capable of infecting human red blood cells is administered to a genetically modified immunodeficient non-human animal, such as a genetically modified immunodeficient mouse of the present invention. The infectious agent may be a *Plasmodium* parasite, such as *Plasmodium falciparum* (*P. falciparum*), *Plasmodium ovale* (*P. ovale*), *Plasmodium vivax* (*P. vivax*), or *Plasmodium malariae* (*P. malariae*).

Optionally, the human red blood cells administered to a genetically modified immunodeficient mouse of the present invention, such as a genetically modified immunodeficient mouse of the present invention, are derived from an individual human or population of human individuals, wherein the individual human or population of human individuals have sickle cell anemia.

In a second aspect, the present invention relates to a method of assaying an effect of a putative therapeutic agent, comprising administering an amount of the putative therapeutic agent to a genetically modified immunodeficient mouse comprising human red blood cells according to the first aspect of the invention and measuring the effect of the putative therapeutic agent.

Otherwise described herein is a method of assaying an effect of a putative therapeutic agent which includes administering an amount of the putative therapeutic agent to a genetically modified immunodeficient non-human animal, wherein the genetically modified immunodeficient non-human animal includes human red blood cells; and measuring the effect of the putative therapeutic agent.

Otherwise described herein is a method of assaying an effect of a putative therapeutic agent is provided which includes administering an amount of the putative therapeutic agent to a genetically modified immunodeficient mouse, wherein the genetically modified immunodeficient mouse includes human red blood cells; and measuring the effect of the putative therapeutic agent.

Abbreviations used for certain genetically modified immunodeficient mouse strains:
MD1: NOD.Cg-*Prkdc^{scid} Il2rg^{tm1Wjl}l*Lyst <emlMvw>/Sz (NSG mouse strain with Lyst knock out by deletion of SEQ ID NO:1 from mouse Lyst gene).
MD2: NSG CD47 KO Tg(hCD47) (NSG mouse strain with mouse CD47 knocked out and including a transgene encoding human CD47).
MD3: NSG CSF1r-HTK (NSG mouse strain including transgene in which the CSFlr promoter drives expression of herpes thymidine kinase).
MD4: B6.129S-Rag1<tm1Mom> CD47 KO Il2rg<tm1Wjl>/Sz ((BL/6 mouse strain with mouse IL2rg knock out and mouse CD47 knock out, also called BL/6 Rag1^{null}CD47KOIL2rg^{null}).

### BRIEF DESCRIPTION OF THE DRAWINGS

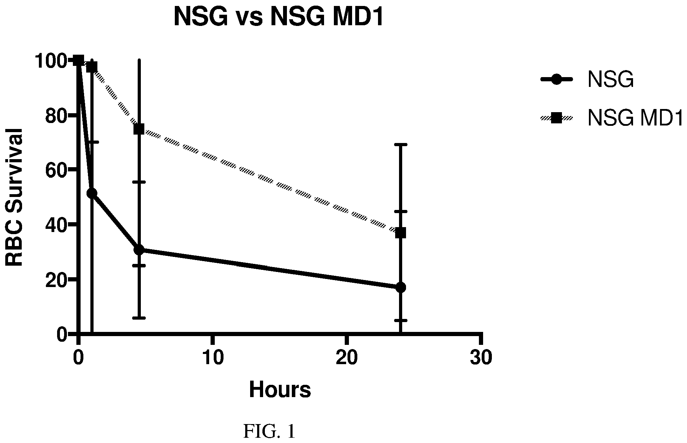

Figure 1 is a graph showing the survival of human red blood cells (RBC) in NSG Lyst (MD1) knock-out (KO) mice compared to NSG control mice and demonstrating a two-fold increase in the number of human red blood cells that survived in the immunodeficient genetically modified mouse at 24 hours after administration of human red blood cells.

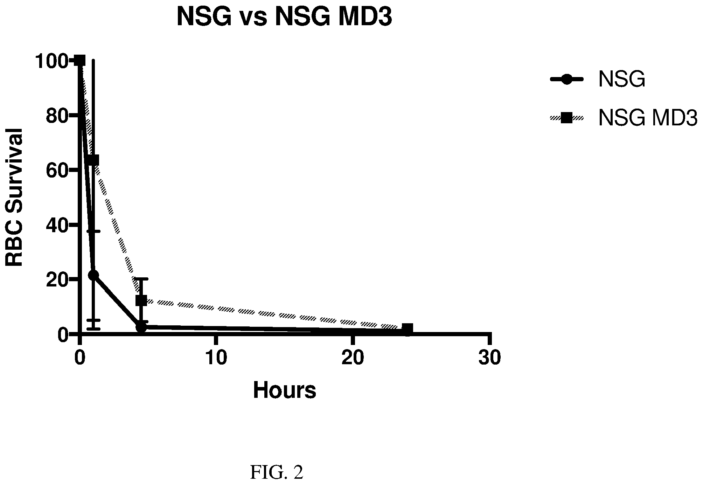

Figure 2 is a graph showing the results from NSG HSV-1-tk transgenic (Tg) mice compared to NSG control mice and demonstrating a significantly higher number of human red blood cells that survived in the immunodeficient genetically modified mouse at up to 24 hours after administration of human red blood cells.

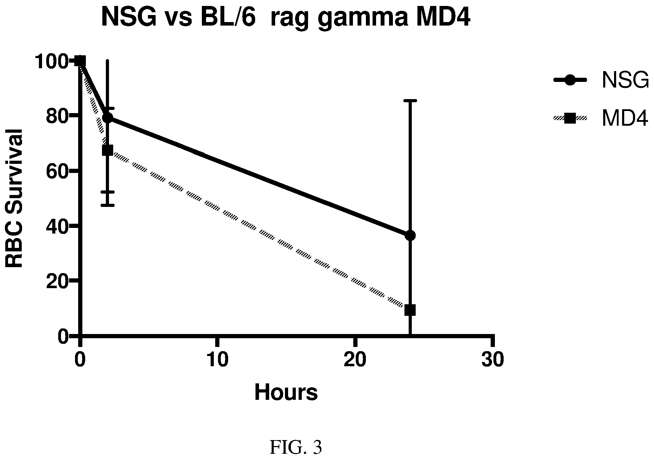

Figure 3 is a graph showing results from BL/6 Rag gamma MD4 mice compared to NSG control mice.

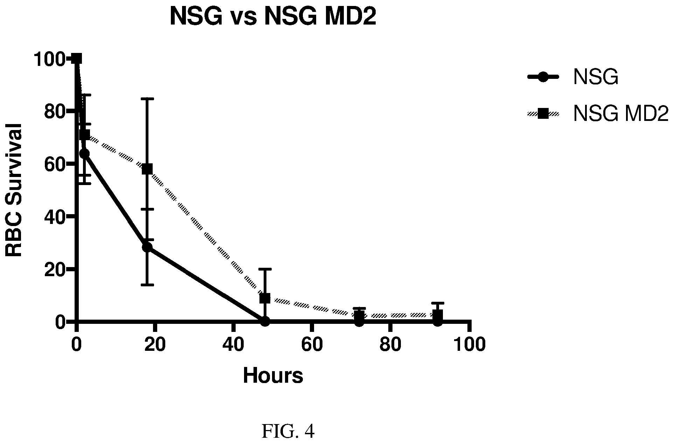

Figure 4 is a graph showing results from NSG MD2 mice compared to NSG control mice and demonstrating a greater than two-fold increase in the number of human red blood cells that survived in the immunodeficient genetically modified mouse at 24 hours after administration of human red blood cells and survival of the human red blood cells in significantly greater numbers in NSG MD2 mice compared to NSG control mice at 48, 72 and 96 hours after administration of the human red blood cells.

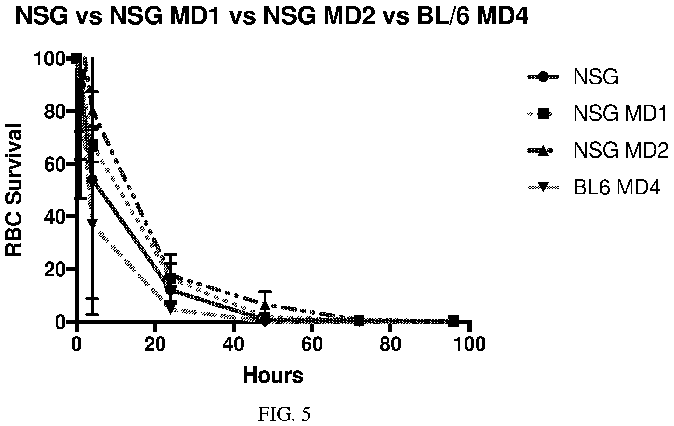

Figure 5 is a graph showing results comparing human RBC survival in several different genetically modified immunodeficient strains according to the first aspect of the present invention compared to NSG control mice and demonstrating an increase in the number of human red blood cells that survived in the immunodeficient genetically modified MD1 and MD2 mice compared to NSG control mice.

### DETAILED DESCRIPTION OF THE INVENTION

Scientific and technical terms used herein are intended to have the meanings commonly understood by those of ordinary skill in the art. Such terms are found defined and used in context in various standard references illustratively including J. Sambrook and D.W. Russell, Molecular Cloning: A Laboratory Manual, Cold Spring Harbor Laboratory Press; 3rd Ed., 2001; F.M. Ausubel, Ed., Short Protocols in Molecular Biology, Current Protocols; 5th Ed., 2002; B. Alberts et al., Molecular Biology of the Cell, 4th Ed., Garland, 2002; D.L. Nelson and M.M. Cox, Lehninger Principles of Biochemistry, 4th Ed., W.H. Freeman & Company, 2004; A. Nagy, M. Gertsenstein, K. Vintersten, R. Behringer, Manipulating the Mouse Embryo: A Laboratory Manual, 3rd edition, Cold Spring Harbor Laboratory Press; December 15, 2002, ISBN-10: 0879695919; Kursad Turksen (Ed.), Embryonic stem cells: methods and protocols in Methods Mol Biol. 2002;185, Humana Press; Current Protocols in Stem Cell Biology, ISBN: 9780470151808; Chu, E. and Devita, V.T., Eds., Physicians' Cancer Chemotherapy Drug Manual, Jones & Bartlett Publishers, 2005; J.M. Kirkwood et al., Eds., Current Cancer Therapeutics, 4th Ed., Current Medicine Group, 2001; Remington: The Science and Practice of Pharmacy, Lippincott Williams & Wilkins, 21st Ed., 2005; L.V. Allen, Jr. et al., Ansel's Pharmaceutical Dosage Forms and Drug Delivery Systems, 8th Ed., Philadelphia, PA: Lippincott, Williams & Wilkins, 2004; and L. Brunton et al., Goodman & Gilman's The Pharmacological Basis of Therapeutics, McGraw-Hill Professional, 12th Ed., 2011.

The singular terms "a," "an," and "the" are not intended to be limiting and include plural referents unless explicitly stated otherwise or the context clearly indicates otherwise.

The present invention is as defined in the appended claims.

Otherwise described herein is a genetically modified immunodeficient non-human animal whose genome includes a genetic modification, wherein the genetic modification renders the non-human animal deficient in macrophages and/or macrophage anti-human red blood cell activity. The genetically modified immunodeficient non-human animal may further include human red blood cells. Human red blood cells administered into the blood system of the genetically modified immunodeficient non-human animal survive longer in the genetically modified immunodeficient non-human animal than in an immunodeficient non-human animal of the same type whose genome does not include the genetic modification.

The term "immunodeficient non-human animal" refers to a non-human animal characterized by one or more of: a lack of functional immune cells, such as T cells and B cells; a DNA repair defect; a defect in the rearrangement of genes encoding antigen-specific receptors on lymphocytes; and a lack of immune functional molecules such as IgM, IgG1, IgG2a, IgG2b, IgG3 and IgA.

Otherwise described herein is a genetically modified immunodeficient non-human animal whose genome includes a genetic modification, wherein the genetic modification renders the non-human animal deficient in macrophages and/or macrophage anti-human red blood cell activity. While description herein describes cases in which the genetically modified immunodeficient non-human animal is a mouse, the genetically modified immunodeficient non-human animal could also be a mammal such as a rat, gerbil, guinea pig, hamster, rabbit, pig, sheep, or non-human primate.

The phrase "genetically modified immunodeficient mouse" as used herein refers to an immunodeficient mouse whose genome includes a genetic modification, wherein the genetic modification renders the immunodeficient mouse deficient in macrophages and/or macrophage anti-human red blood cell activity.

The phrase "deficient in macrophages" refers to a reduction in the number of macrophages compared to the number of macrophages present in a comparable immunodeficient mouse which does not have the genetic mutation that renders the immunodeficient mouse deficient in macrophages.

The term "immunodeficient mouse" refers to a mouse characterized by one or more of: a lack of functional immune cells, such as T cells and B cells; a DNA repair defect; a defect in the rearrangement of genes encoding antigen-specific receptors on lymphocytes; and a lack of immune functional molecules such as IgM, IgG1, IgG2a, IgG2b, IgG3 and IgA. Immunodeficient mice can be characterized by one or more deficiencies in a gene involved in immune function, such as *Rag1* and *Rag2* (Oettinger, M.A et al., Science, 248:1517-1523, 1990; and Schatz, D. G. et al., Cell, 59:1035-1048, 1989) Immunodeficient mice may have any of these or other defects which result in abnormal immune function in the mice.

Particularly useful immunodeficient mouse strains are NOD.Cg-*Prkdc^{scid} Il2rg^{tm1Wjl}*/SzJ*,* commonly referred to as NOD scid gamma (NSG) mice, described in detail in Shultz LD et al, 2005, J. Immunol, 174:6477-89; NOD.Cg-*Rag1tm1Mom Il2rg^{tm1Wjl}*/SzJ*,* Shultz LD et al, 2008 Clin Exp Immunol 154(2):270-84 commonly referred to as NRG mice; and NOD.Cg-*Prkdc^{scid} Il2rg^{tm1Sug}l*JicTac*,* described in detail in Ito, M. et al., Blood 100, 3175-3182 (2002) commonly referred to as NOG mice.

The term "severe combined immune deficiency (SCID)" refers to a condition characterized by absence of T cells and lack of B cell function.

Common forms of SCID include: X-linked SCID which is characterized by gamma chain gene mutations in the *IL2RG* gene and the lymphocyte phenotype T(-) B(+) NK(-); and autosomal recessive SCID characterized by Jak3 gene mutations and the lymphocyte phenotype T(-) B(+) NK(-), ADA gene mutations and the lymphocyte phenotype T(-) B(-) NK(-), IL-7R alpha-chain mutations and the lymphocyte phenotype T(-) B(+) NK(+), CD3 delta or epsilon mutations and the lymphocyte phenotype T(-) B(+) NK(+), RAG1/RAG2 mutations and the lymphocyte phenotype T(-) B(-) NK(+), Artemis gene mutations and the lymphocyte phenotype T(-) B(-) NK(+), CD45 gene mutations and the lymphocyte phenotype T(-) B(+) NK(+).

In further aspects, a genetically modified immunodeficient mouse has a defect in its endogenous gene encoding DNA-dependent protein kinase, catalytic subunit (Prkdc) which causes the mouse to express a defective endogenous DNA-dependent protein kinase, catalytic subunit and/or a reduced amount of endogenous DNA-dependent protein kinase, catalytic subunit, or the mouse may not express endogenous DNA-dependent protein kinase, catalytic subunit at all. The immunodeficient mouse can optionally be Prkdc null such that it lacks a functional endogenous *Prkdc* gene).

A genetically modified mouse according to aspects of the present invention has the severe combined immunodeficiency mutation *(Prkdc^{scid}),* commonly referred to as the *scid* mutation. The *scid* mutation is well-known and located on mouse chromosome 16 as described in Bosma, et al., Immunogenetics 29:54-56, 1989. Mice homozygous for the *scid* mutation are characterized by an absence of functional T cells and B cells, lymphopenia, hypoglobulinemia and a normal hematopoetic microenvironment. The *scid* mutation can be detected, for example, by detection of markers for the *scid* mutation using well-known methods, such as PCR or flow cyotometry.

Otherwise described herein is a genetically modified mouse having an IL2 receptor gamma chain deficiency. The term "IL2 receptor gamma chain deficiency" refers to decreased IL2 receptor gamma chain. Decreased IL2 receptor gamma chain can be due to gene deletion or mutation. Decreased IL2 receptor gamma chain can be detected, for example, by detection of IL2 receptor gamma chain gene deletion or mutation and/or detection of decreased IL2 receptor gamma chain expression using well-known methods.

According to the first aspect of the present invention, a genetically modified immunodeficient NSG mouse is provided whose genome includes a genetic modification, wherein the genetic modification renders the mice deficient in macrophages and/or macrophage anti-human red blood cell activity.

According to the first aspect of the present invention, a genetically modified immunodeficient NRG mouse is provided whose genome includes a genetic modification, wherein the genetic modification renders the mice deficient in macrophages and/or macrophage anti-human red blood cell activity.

According to the first aspects of the present invention, a genetically modified immunodeficient NOG mouse is provided whose genome includes a genetic modification, wherein the genetic modification renders the mice deficient in macrophages and/or macrophage anti-human red blood cell activity.

### Genetic modification that produces an immunodeficient mouse deficient in macrophages and/or macrophage anti-human red blood cell activity

According to the first aspect of the present invention, a genetically modified immunodeficient mouse is provided wherein the genetic modification is a mutation of a lysosomal trafficking regulator (Lyst) gene such that the mouse does not express functional lysosomal trafficking regulator protein rendering the non-human animal deficient in macrophages and/or macrophage anti-human red blood cell activity. The genetically modified immunodeficient mice further include human red blood cells according to the first aspect of the present invention. Human red blood cells administered into the blood system of the genetically modified immunodeficient non-human animals survive longer in the genetically modified immunodeficient non-human animals than in immunodeficient non-human animals of the same type whose genome does not include the genetic modification. The Lyst gene is located at Chr13:13590409-13777440 bp, + strand in mouse and is conserved in numerous species including human, chimpanzee, Rhesus monkey, dog, cow, rat, chicken, zebrafish, and frog.

Otherwise described herein is a genetically modified immunodeficient mouse having one or more spontaneous or induced mutations at the Lyst locus are provided wherein the mouse does not express functional lysosomal trafficking regulator protein rendering the mouse deficient in macrophages and/or macrophage anti-human red blood cell activity. The genetically modified immunodeficient mouse may further include human red blood. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient mouse having a deletion in exon 5 at the Lyst locus is provided wherein the mouse does not express functional lysosomal trafficking regulator protein rendering the mouse deficient in macrophages and/or macrophage anti-human red blood cell activity. The genetically modified immunodeficient mouse may further include human red blood cells. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient mouse having a 25 bp deletion in exon 5 at the Lyst locus is provided wherein the mouse does not express functional lysosomal trafficking regulator protein rendering the mouse deficient in macrophages and/or macrophage anti-human red blood cell activity. The 25 bp deletion could be deletion of a 25 bp segment of genomic DNA in exon 5 at the Lyst locus having the nucleotide sequence GAGCCGGTAGCTTTGGTTCAACGGA (SEQ ID NO: 1). The genetically modified immunodeficient mouse may further includes human red blood cells Human red blood cells administered into the blood system of the genetically modified immunodeficient mouse survive longer in the genetically modified immunodeficient mouse than in an immunodeficient mouse of the same type whose genome does not include the genetic modification.

According to the first aspect of the present invention, a genetically modified immunodeficient mouse having a mutation at the Lyst locus such that the mice are Lyst^{null} is provided wherein the mouse does not express functional lysosomal trafficking regulator protein rendering the mouse deficient in macrophages and/or macrophage anti-human red blood cell activity. The genetically modified immunodeficient mouse further includes human red blood cells according to the first aspect of the present invention. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient mouse homozygous for a beige mutation Lyst^{bg} is provided wherein the mouse does not express functional lysosomal trafficking regulator protein rendering the mouse deficient in macrophages and/or macrophage anti-human red blood cell activity. A Lyst^{bg} remutation (*Lystbg-J*) occurred spontaneously in the C57BL/6J strain at The Jackson Laboratory (J:5311). This allele is defined by a noncomplementation test with Lyst^{bg.} This is the result of a 3 bp deletion in exon 54 of *Lyst* causing an isoleucine deletion at codon 3741 near the carboxy terminus of the protein. This deletion affects the WD40 domain. The genetically modified immunodeficient mouse further may include human red blood cells. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient NOD.Cg-*Prkdc^{scid} Il2rg^{tm1Wjl}l*SzJ mouse homozygous for a beige mutation Lyst^{bg} wherein the mouse does not express functional lysosomal trafficking regulator protein rendering the mouse deficient in macrophages and/or macrophage anti-human red blood cell activity. The genetically modified immunodeficient mouse may further include human red blood cells. Human red blood cells administered into the blood system of the genetically modified immunodeficient mouse survive longer in the genetically modified immunodeficient mouse than in an immunodeficient mouse of the same type whose genome does not include the genetic modification.

According to the first aspect of the present invention, a genetically modified immunodeficient NOD. Cg-*Prkdc^{scid} Il2rg^{tm1Wjl}l*Lyst <emlMvw>/Sz (NSG Lyst knock out - MD1) mouse is provided wherein the mouse does not express functional lysosomal trafficking regulator protein rendering the mouse deficient in macrophages and/or macrophage anti-human red blood cell activity. The genetically modified immunodeficient mouse further includes human red blood cells according to the first aspect of the present invention. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient NOD.Cg-*Rag1^{tm1Mom} Il2rg^{tm1Wjl}l*SzJ mouse homozygous for a beige mutation Lyst^{bg} wherein the mouse does not express functional lysosomal trafficking regulator protein rendering the mouse deficient in macrophages and/or macrophage anti-human red blood cell activity. The genetically modified immunodeficient mouse may further include human red blood cells. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient mouse wherein the genetic modification includes a transgene encoding human CD47 such that the mouse expresses human CD47 protein and further includes a mutation of the mouse CD47 gene in the genome of the mouse such that the mouse does not express functional mouse CD47 protein, rendering the mouse deficient in macrophages and/or macrophage anti-human red blood cell activity. The genetically modified immunodeficient mouse may further include human red blood cells. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient NSG mouse, wherein the genetic modification includes a transgene encoding human CD47 such that the mouse expresses human CD47 protein and further includes a mutation of the mouse CD47 gene in the genome of the mouse such that the mouse does not express functional mouse CD47 protein, rendering the mouse deficient in macrophages and/or macrophage anti-human red blood cell activity. The genetically modified immunodeficient mouse may further include human red blood cells. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient NRG mouse, wherein the genetic modification includes a transgene encoding human CD47 such that the mouse expresses human CD47 protein and further includes a mutation of the mouse CD47 gene in the genome of the mouse such that the mouse does not express functional mouse CD47 protein, rendering the mouse deficient in macrophages and/or macrophage anti-human red blood cell activity. The genetically modified immunodeficient mouse may further include human red blood cells. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient NOG mouse, wherein the genetic modification includes a transgene encoding human CD47 such that the mouse expresses human CD47 protein and further includes a mutation of the mouse CD47 gene in the genome of the mouse such that the mouse does not express functional mouse CD47 protein, rendering the mouse deficient in macrophages and/or macrophage anti-human red blood cell activity. The genetically modified immunodeficient mouse may further include human red blood cells. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient NOD.Cg-Prkdc<scid> Cd47<tm1Fpl> Il2rg<tm1Wjl> Tg(CD47)2Sz/Sz (NSG Cd47 KO human CD47 Tg) mouse. The genetically modified immunodeficient mouse may further include human red blood cells. Human red blood cells administered into the blood system of the genetically modified immunodeficient mouse survive longer in the genetically modified immunodeficient mouse than in an immunodeficient mouse of the same type whose genome does not include the genetic modification.

Otherwise described herein is a genetically modified immunodeficient mouse, wherein the genetic modification includes a transgene encoding herpes simplex virus 1 thymidine kinase such that the mouse expresses herpes simplex virus 1 thymidine kinase protein which, in combination with a nucleoside analog, renders the mouse deficient in macrophages. Any nucleoside analog which is toxic to macrophages in combination with herpes simplex virus 1 thymidine kinase protein can be used, exemplified by, but not limited to, ganciclovir, acyclovir or a combination thereof. The genetically modified immunodeficient mouse may further include human red blood cells. Human red blood cells administered into the blood system of the genetically modified immunodeficient mouse survive longer in the genetically modified immunodeficient mouse than in an immunodeficient mouse of the same type whose genome does not include the genetic modification.

Any of various methods can be used to produce a genetically modified immunodeficient non-human animal, such as a mouse, whose genome includes a genetic modification. Genetic modifications are produced using standard methods of genetic engineering such as, but not limited to, chemical mutagenesis, irradiation, homologous recombination and transgenic expression of antisense RNA. Such techniques are well-known in the art and further include, but are not limited to, pronuclear microinjection and transformation of embryonic stem cells. Methods for generating genetically modified animals whose genome includes a disrupted gene that can be used include, but are not limited to, those described in J. P. Sundberg and T. Ichiki, Eds., Genetically Engineered Mice Handbook, CRC Press; 2006; M. H. Hofker and J. van Deursen, Eds., Transgenic Mouse Methods and Protocols, Humana Press, 2002; A. L. Joyner, Gene Targeting: A Practical Approach, Oxford University Press, 2000; Manipulating the Mouse Embryo: A Laboratory Manual, 3rd edition, Cold Spring Harbor Laboratory Press; December 15, 2002, ISBN-10: 0879695919; Kursad Turksen (Ed.), Embryonic stem cells: methods and protocols in Methods Mol Biol. 2002;185, Humana Press; Current Protocols in Stem Cell Biology, ISBN: 978047015180; Meyer et al. PNAS USA, vol. 107 (34), 15022-15026.

Homology-based recombination gene modification strategies can be used to genetically modify an immunodeficient mouse by "knock-in" to introduce a nucleic acid encoding an exogenous protein or proteins e.g., a nucleotide sequence encoding herpes simplex virus 1 thymidine kinase or a nucleotide sequence encoding human CD47 into the genome of the immunodeficient mouse. Similarly, a homology-based recombination gene modification strategy can be used to genetically modify an immunodeficient mouse by "knock-out" or mutate a gene encoding an enogenous protein or proteins e.g., mouse CD47 or mouse Lyst.

Homology-based recombination gene modification strategies include gene editing approaches such as those using homing endonucleases, integrases, meganucleases, transposons, nuclease-mediated processes using a zinc finger nuclease (ZFN), a Transcription Activator-Like (TAL), a Clustered Regularly Interspaced Short Palindromic Repeats (CRISPR)-Cas, or a Drosophila Recombination-Associated Protein (DRAP) approach. See, for example, Cerbini et al., PLoS One. 2015; 10(1): e0116032; Shen et al., PLoS ONE 8(10): e77696; and Wang et al., Protein & Cell, February 2016, Volume 7, Issue 2, pp 152-156.

Genomic editing is performed, for example, by methods described herein, and as detailed in J. P. Sundberg and T. Ichiki, Eds., Genetically Engineered Mice Handbook, CRC Press; 2006; M. H. Hofker and J. van Deursen, Eds., Transgenic Mouse Methods and Protocols, Humana Press, 2002; A. L. Joyner, Gene Targeting: A Practical Approach, Oxford University Press, 2000; Manipulating the Mouse Embryo: A Laboratory Manual, 3rd edition, Cold Spring Harbor Laboratory Press; December 15, 2002, ISBN-10: 0879695919; Kursad Turksen (Ed.), Embryonic stem cells: methods and protocols in Methods Mol Biol. 2002;185, Humana Press; Current Protocols in Stem Cell Biology, ISBN: 978047015180; Meyer et al., PNAS USA, 2010, vol. 107 (34), 15022-15026; and Doudna, J. et al. (eds.) CRISPR-Cas: A Laboratory Manual, 2016, CSHP. A brief description of several genomic editing techniques is described herein.

### Nuclease Techniques for Genetic Modification of Mice

A genetic modification method, such as but not limited to, a nuclease genetic editing technique, can be used to introduce a desired DNA sequence into the genome at a predetermined target site, such as methods using a homing endonuclease, integrase, meganuclease, transposon, nuclease-mediated process using a zinc finger nuclease (ZFN), a Transcription Activator-Like (TAL), a Clustered Regularly Interspaced Short Palindromic Repeats (CRISPR)-Cas, or Drosophila Recombination-Associated Protein (DRAP). Briefly, a genetic modification method that can be used includes introducing into an ES cell, iPS cell, somatic cell, fertilized oocyte or embryo, RNA molecules encoding a targeted TALEN, ZFN, CRISPR or DRAP and at least one oligonucleotide, then selecting for an ES cell, iPS cell, somatic cell, fertilized oocyte or embryo with the desired genetic modification.

For example, a desired nucleic acid sequence can be introduced into the genome of a mouse at a predetermined target site by a nuclease technique, such as, but not limited to, CRISPR methodology, TAL (transcription activator-like Effector methodology, Zinc Finger-Mediated Genome Editing or DRAP to produce a genetically modified mouse described herein whose genome includes a nucleic acid encoding human CD47 or herpes simplex virus 1 thymidine kinase protein operably linked to a promoter, wherein the animal expresses the encoded human CD47 or herpes simplex virus 1 thymidine kinase protein.

As used herein, the terms "target site" and "target sequence" in the context of a nuclease genetic editing technique refer to a nucleic acid sequence that defines a portion of a chromosomal sequence to be edited and to which a nuclease is engineered to recognize and bind, provided sufficient conditions for binding exist.

### CRISPR-Cas System

CRISPRs (Clustered Regularly Interspaced Short Palindromic Repeats) are loci containing multiple short direct repeats that are found in the genomes of approximately 40% of sequenced bacteria and 90% of sequenced archaea and confer resistance to foreign DNA elements, see Horvath, 2010, Science, 327: 167-170; Barrangou et al, 2007, Science, 315: 1709-1712; and Makarova et al, 2011, Nature Reviews Microbiology. 9: 467-477.

CRISPR repeats range in size from 24 to 48 base pairs. They usually show some dyad symmetry, implying the formation of a secondary structure such as a hairpin, but are not truly palindromic. CRISPR repeats are separated by spacers of similar length.

The CRISPR-associated (cas) genes are often associated with CRISPR repeat-spacer arrays. More than forty different Cas protein families have been described (Haft et al. 2005, PLoS Comput Biol. 1 (6): e60). Particular combinations of cas genes and repeat structures have been used to define 8 CRISPR subtypes, some of which are associated with an additional gene module encoding repeat-associated mysterious proteins (RAMPs).

There are diverse CRISPR systems in different organisms, and one of the simplest is the type II CRISPR system from Streptococcus pyogenes: only a single gene encoding the Cas9 protein and two RNAs, a mature CRISPR RNA (crRNA) and a partially complementary trans-acting RNA (tracrRNA), are necessary and sufficient for RNA-guided silencing of foreign DNAs (Gasiunas et al, 2012, PNAS 109: E2579-E2586; Jinek et al, 2012, Science 337: 816-821). Maturation of crRNA requires tracrRNA and RNase III (Deltcheva et al, 2011, Nature 471: 602-607). However, this requirement can be bypassed by using an engineered small guide RNA (sgRNA) containing a designed hairpin that mimics the tracrRNA-crRNA complex (Jinek et al., 2012, Science 337: 816-821). Base pairing between the sgRNA and target DNA causes double-strand breaks (DSBs) due to the endonuclease activity of Cas9. Binding specificity is determined by both sgRNA-DNA base pairing and a short DNA motif (protospacer adjacent motif [PAM] sequence: NGG) juxtaposed to the DNA complementary region (Marraffini & Sontheimer, 2010, Nature Reviews Genetics, 11: 181-190). For example, the CRISPR system requires a minimal set of two molecules, the Cas9 protein and the sgRNA, and therefore can be used as a host-independent gene-targeting platform. The Cas9/CRISPR can be harnessed for site-selective RNA-guided genome editing, such as targeting insertion see for example, Carroll, 2012, Molecular Therapy 20: 1658-1660; Chang et al, 2013, Cell Research 23: 465-472; Cho et al, 2013, Nature Biotechnol 31: 230-232; Cong et al, 2013, Science 339: 819-823; Hwang et al, 2013, Nature Biotechnol 31: 227-229; Jiang et al, 2013, Nature Biotechnol 31: 233-239; Mali et al, 2013, Science 339: 823-826; Qi et al, 2013, Cell 152: 1173-1183; Shen et al, 2013, Cell Research 23: 720-723; and Wang et al, 2013, Cell 153: 910-918). In particular, Wang et al. 2013, Cell 153: 910-918 describe targeted insertion using the CRISPR/Cas9 system combined with oligonucleotides.

Generation of a genetically modified mouse according to the first aspect of the present invention may include injection or transfection of appropriate nucleic acids, such as an expression construct encoding cas9 and an expression construct encoding a guide RNA specific for the gene to be targeted, for use in CRISPR, into a preimplantation embryo or stem cells, such as embryonic stem (ES) cells or induced pluripotent stem (iPS) cells. Optionally, cas9 and the guide RNA are encoding in a single expression construct.

### TAL (transcription activator-like) Effectors

Transcription activator-like (TAL) effectors or TALE (transcription activator-like effector) are derived from a plant pathogenic bacteria genus, Xanthomonas, and these proteins mimic plant transcriptional activators and manipulate the plant transcript, see Kay et al., 2007, Science, 318:648-651.

TAL effectors contain a centralized domain of tandem repeats, each repeat containing approximately 34 amino acids, which are key to the DNA binding specificity of these proteins. In addition, they contain a nuclear localization sequence and an acidic transcriptional activation domain, for a review see Schornack et al 2006, J. Plant Physiol., 163(3): 256-272; Scholze and Boch, 2011, Curr Opin Microbiol, 14:47-53.

Specificity of TAL effectors depends on the sequences found in the tandem repeats. The repeated sequence includes approximately 102 bp and the repeats are typically 91-100% homologous with each other (Bonas et al, 1989, Mol Gen Genet 218: 127-136). Polymorphism of the repeats is usually located at positions 12 and 13 and there appears to be a one-to-one correspondence between the identity of the hypervariable diresidues at positions 12 and 13 with the identity of the contiguous nucleotides in the TAL-effector's target sequence, see Moscou and Bogdanove 2009, Science 326: 1501; and Boch et al 2009, Science 326:1509-1512. The two hypervariable residues are known as repeat variable diresidues (RVDs), whereby one RVD recognizes one nucleotide of DNA sequence and ensures that the DNA binding domain of each TAL-effector can target large recognition sites with high precision (15 - 30nt). Experimentally, the code for DNA recognition of these TAL-effectors has been determined such that an HD sequence at positions 12 and 13 leads to a binding to cytosine (C), NG binds to T, NI to A, C, G or T, NN binds to A or G, and IG binds to T. These DNA binding repeats have been assembled into proteins with new combinations and numbers of repeats, to make artificial transcription factors that are able to interact with new sequences and activate the expression of a reporter gene in plant cells (Boch et al 2009, Science 326:1509-1512). These DNA binding domains have been shown to have general applicability in the field of targeted genomic editing or targeted gene regulation in all cell types, see Gaj et al., Trends in Biotechnol, 2013, 31(7):397-405. Moreover, engineered TAL effectors have been shown to function in association with exogenous functional protein effector domains such as a nuclease, not naturally found in natural Xanthomonas TAL-effect or proteins in mammalian cells. TAL nucleases (TALNs or TALENs) can be constructed by combining TALs with a nuclease, e.g. Fokl nuclease domain at the N-terminus or C-terminus, Kim et al. 1996, PNAS 93:1156-1160; Christian et al 2010, Genetics 186:757-761; Li et al, 2011, Nucleic Acids Res 39: 6315-6325; and Miller et al, 2011, Nat Biotechnol 29: 143-148. The functionality of TALENs to cause deletions by NHEJ has been shown in rat, mouse, zebrafish, Xenopus, medaka, rat and human cells, Ansai et al, 2013, Genetics, 193: 739-749; Carlson et al, 2012, PNAS, 109: 17382-17387; Hockemeyer et al, 2011, Nature Biotechnol., 29: 731-734; Lei et al, 2012, PNAS, 109: 17484-17489; Moore et al, 2012, PLoS ONE, 7: e37877; Stroud et al, 2013, J. Biol. Chem., 288: 1685-1690; Sung et al, 2013, Nature Biotechnol 31: 23-24; Wefers et al, 2013, PNAS 110: 3782-3787.

For TALEN, methods of making such are further described in the US patents US8420782, US8450471, US8450107, US8440432, US8440431 and US patent applications US20130137161, US20130137174.

Other useful endonucleases may include, for example, Hhal, HindIII, NotI, BbvCI, EcoRI, Bg/I, and AlwI. The fact that some endonucleases (e.g., Fokl) only function as dimers can be capitalized upon to enhance the target specificity of the TAL effector. For example, in some cases each Fokl monomer can be fused to a TAL effector sequence that recognizes a different DNA target sequence, and only when the two recognition sites are in close proximity do the inactive monomers come together to create a functional enzyme. By requiring DNA binding to activate the nuclease, a highly site-specific restriction enzyme can be created.

In some embodiments, the TALEN may further include a nuclear localization signal or sequence (NLS). A NLS is an amino acid sequence that facilitates targeting the TALEN nuclease protein into the nucleus to introduce a double stranded break at the target sequence in the chromosome.

Nuclear localization signals are known in the art, see, for example, Makkerh et al. 1996, Curr Biol. 6:1025-1027. NLS include the sequence PKKKRKV from SV40 Large T-antigen, Kalderon 1984, Cell, 39: 499-509; RPAATKKAGQAKKK (SEQ ID NO: 9) from nucleoplasmin, Dingwallet al., 1988, J Cell Biol., 107, 841-9. Further examples are described in McLane and Corbett 2009, IUBMB Life, 61, 697-70; Dopie et al. 2012, PNAS, 109, E544-E552.

The cleavage domain may be obtained from any endonuclease or exonuclease. Non-limiting examples of endonucleases from which a cleavage domain may be derived include, but are not limited to, restriction endonucleases and homing endonucleases. See, for example, 2002-2003 Catalog, New England Biolabs, Beverly, Mass.; and Belfort et al. (1997) Nucleic Acids Res. 25:3379-3388. Additional enzymes that cleave DNA are known, e.g., SI Nuclease; mung bean nuclease; pancreatic DNase I; micrococcal nuclease; yeast HO endonuclease. See also Linn et al. (eds.) Nucleases, Cold Spring Harbor Laboratory Press, 1993. One or more of these enzymes, or functional fragments thereof, may be used as a source of cleavage domains.

### Zinc Finger-Mediated Genome Editing

The use of zinc finger nucleases (ZFN) for gene editing, such as for targeted insertion via a homology-directed repair process, has been well established. For example see Carbery et al, 2010, Genetics, 186: 451-459; Cui et al, 2011, Nature Biotechnol., 29: 64-68; Hauschild et al, 2011, PNAS, 108: 12013-12017; Orlando et al, 2010, Nucleic Acids Res., 38: e152-e152; and Porteus & Carroll, 2005, Nature Biotechnology, 23: 967-973.

Components of the ZFN-mediated process include a zinc finger nuclease with a DNA binding domain and a cleavage domain. Such are described for example in Beerli et al. (2002) Nature Biotechnol., 20:135-141; Pabo et al. (2001) Ann. Rev. Biochem., 70:313-340; Isalan et al. (2001) Nature Biotechnol. 19:656-660; Segal et al. (2001) Curr Opin. Biotechnol., 12:632-637; and Choo et al. (2000) Curr Opin. Struct. Biol., 10:411-416; and U.S. Pat. Nos. 6,453,242 and 6,534,261. Methods to design and select a zinc finger binding domain to a target sequence are known in the art, see for example Sera, et al., Biochemistry 2002,41,7074-7081; U.S. Pat. Nos. 6,607,882; 6,534,261 and 6,453,242.

In some embodiments, the zinc finger nuclease may further include a nuclear localization signal or sequence (NLS). A NLS is an amino acid sequence that facilitates targeting the zinc finger nuclease protein into the nucleus to introduce a double stranded break at the target sequence in the chromosome. Nuclear localization signals are known in the art. See, for example, Makkerh et al. (1996) Current Biology 6:1025-1027.

The cleavage domain may be obtained from any endonuclease or exonuclease. Non-limiting examples of endonucleases from which a cleavage domain may be derived include, but are not limited to, restriction endonucleases and homing endonucleases. See, for example, 2002-2003 Catalog, New England Biolabs, Beverly, Mass.; and Belfort et al. (1997) Nucleic Acids Res. 25:3379-3388. Additional enzymes that cleave DNA are known (e.g., SI Nuclease; mung bean nuclease; pancreatic DNase I; micrococcal nuclease; yeast HO endonuclease). See also Linn et al. (eds.) Nucleases, Cold Spring Harbor Laboratory Press, 1993. One or more of these enzymes (or functional fragments thereof) may be used as a source of cleavage domains. A cleavage domain also may be derived from an enzyme or portion thereof, as described above, that requires dimerization for cleavage activity.

Two zinc finger nucleases may be required for cleavage, as each nuclease includes a monomer of the active enzyme dimer. Alternatively, a single zinc finger nuclease may include both monomers to create an active enzyme dimer. Restriction endonucleases (restriction enzymes) are present in many species and are capable of sequence-specific binding to DNA (at a recognition site), and cleaving DNA at or near the site of binding. Certain restriction enzymes (e.g., Type IIS) cleave DNA at sites removed from the recognition site and have separable binding and cleavage domains. For example, the Type IIS enzyme Fokl catalyzes double stranded cleavage of DNA, at 9 nucleotides from its recognition site on one strand and 13 nucleotides from its recognition site on the other. See, for example, U.S. Pat. Nos. 5,356,802; 5,436,150 and 5,487,994; as well as Li et al. (1992) PNAS 89:4275-4279; Li et al. (1993) PNAS 90:2764-2768; Kim et al. (1994) PNAS 91:883-887; Kim et al. (1994) J. Biol. Chem. 269:31, 978-31, 982. Thus, a zinc finger nuclease may include the cleavage domain from at least one Type IIS restriction enzyme and one or more zinc finger binding domains, which may or may not be engineered. Exemplary Type IIS restriction enzymes are described for example in International Publication WO 07/014275,. Additional restriction enzymes also contain separable binding and cleavage domains, and these also are contemplated by the present disclosure. See, for example, Roberts et al. (2003) Nucleic Acids Res. 31: 418-420. An exemplary Type IIS restriction enzyme, whose cleavage domain is separable from the binding domain, is FokI. This particular enzyme is active as a dimer (Bitinaite et al. 1998, PNAS 95: 10,570-10,575). Accordingly, for the purposes of the present disclosure, the portion of the Fokl enzyme used in a zinc finger nuclease is considered a cleavage monomer. Thus, for targeted double stranded cleavage using a Fokl cleavage domain, two zinc finger nucleases, each including a Fokl cleavage monomer, may be used to reconstitute an active enzyme dimer. Alternatively, a single polypeptide molecule containing a zinc finger binding domain and two Fokl cleavage monomers may also be used. In certain embodiments, the cleavage domain may include one or more engineered cleavage monomers that minimize or prevent homodimerization, as described, for example, in U.S. Patent Publication Nos. 20050064474, 20060188987, and 20080131962,. By way of non-limiting example, amino acid residues at positions 446, 447, 479, 483, 484, 486, 487, 490, 491, 496, 498, 499, 500, 531, 534, 537 and 538 of Fokl are all targets for influencing dimerization of the Fokl cleavage half-domains. Exemplary engineered cleavage monomers of Fokl that form obligate heterodimers include a pair in which a first cleavage monomer includes mutations at amino acid residue positions 490 and 538 of Fokl and a second cleavage monomer that includes mutations at amino-acid residue positions 486 and 499. Thus, in one embodiment, a mutation at amino acid position 490 replaces Glu (E) with Lys (K); a mutation at amino acid residue 538 replaces Ile (I) with Lys (K); a mutation at amino acid residue 486 replaces Gln (Q) with Glu (E); and a mutation at position 499 replaces Ile (I) with Lys (K). Specifically, the engineered cleavage monomers may be prepared by mutating positions 490 from E to K and 538 from I to K in one cleavage monomer to produce an engineered cleavage monomer designated "E490K:I538K" and by mutating positions 486 from Q to E and 499 from I to L in another cleavage monomer to produce an engineered cleavage monomer designated "Q486E:I499L." The above described engineered cleavage monomers are obligate heterodimer mutants in which aberrant cleavage is minimized or abolished. Engineered cleavage monomers may be prepared using a suitable method, for example, by site-directed mutagenesis of wild-type cleavage monomers (Fokl) as described in U.S. Patent Publication No. 20050064474.

The zinc finger nuclease described above may be engineered to introduce a double stranded break at the targeted site of integration. The double stranded break may be at the targeted site of integration, or it may be up to 1, 2, 3, 4, 5, 10, 15, 20, 25, 30, 35, 40, 45, 50, 100 or 1000 nucleotides away from the site of integration. In some embodiments, the double stranded break may be up to 1, 2, 3, 4, 5, 10, 15, or 20 nucleotides away from the site of integration. In other embodiments, the double stranded break may be up to 10, 15, 20, 25, 30, 35, 40, 45 or 50 nucleotides away from the site of integration. In yet other embodiments, the double stranded break may be up to 50, 100 or 1000 nucleotides away from the site of integration.

The DRAP technology has been described in US6534643, US6858716 and US6830910 and Watt et al, 2006.

Generation of a genetically modified immunodeficient mouse whose genome includes a genetic modification, wherein the genetic modification renders the mouse deficient in macrophages and/or macrophage anti-human red blood cell activity can be achieved by introduction of a gene targeting vector into a preimplantation embryo or stem cells, such as embryonic stem (ES) cells or induced pluripotent stem (iPS) cells.

The term "gene targeting vector" refers to a double-stranded recombinant DNA molecule effective to recombine with and mutate a specific chromosomal locus, such as by insertion into or replacement of the targeted gene.

For targeted gene disruption or introduction of a desired nucleic acid sequence, a gene targeting vector is made using recombinant DNA techniques and includes 5' and 3' sequences which are homologous to the stem cell endogenous target gene. The gene targeting vector optionally and preferably further includes a selectable marker such as neomycin phosphotransferase, hygromycin or puromycin. Those of ordinary skill in the art are capable of selecting sequences for inclusion in a gene targeting vector and using these with no more than routine experimentation. Gene targeting vectors can be generated recombinantly or synthetically using well-known methodology.

For methods of DNA injection of a gene targeting vector into a preimplantation embryo, the gene targeting vector is linearized before injection into non-human preimplantation embryos. Preferably, the gene targeting vector is injected into fertilized oocytes. Fertilized oocytes are collected from superovulated females the day after mating (0.5 dpc) and injected with the expression construct. The injected oocytes are either cultured overnight or transferred directly into oviducts of 0.5-day p.c. pseudopregnant females. Methods for superovulation, harvesting of oocytes, gene targeting vector injection and embryo transfer are known in the art and described in Manipulating the Mouse Embryo: A Laboratory Manual, 3rd edition, Cold Spring Harbor Laboratory Press; December 15, 2002, ISBN-10: 0879695919. Offspring can be tested for the presence of target gene disruption by DNA analysis, such as PCR, Southern blot or sequencing. Mice having disrupted target gene can be tested for target protein expression such as by using ELISA or Western blot analysis and/or mRNA expression such as by RT-PCR.

Alternatively the gene targeting vector may be transfected into stem cells (ES cells or iPS cells) using well-known methods, such as electroporation, calcium-phosphate precipitation and lipofection.

Mouse ES cells are grown in media optimized for the particular line. Typically ES media contains 15% fetal bovine serum (FBS) or synthetic or semisynthetic equivalents, 2 mM glutamine, 1 mM Na Pyruvate, 0.1 mM non-essential amino acids, 50 U/ml penicillin and streptomycin, 0.1 mM 2-mercaptoethanol and 1000 U/ml LIF (plus, for some cell lines chemical inhibitors of differentiation) in Dulbecco's Modified Eagle Media (DMEM). A detailed description is known in the art (Tremml et al., 2008, Current Protocols in Stem Cell Biology, Chapter 1:Unit 1C.4. For review of inhibitors of ES cell differentiation, see Buehr, M.,et al. (2003). Genesis of embryonic stem cells. Philosophical Transactions of the Royal Society B: Biological Sciences 358, 1397-1402.

The cells are screened for target gene disruption or introduction of a desired nucleic acid sequence by DNA analysis, such as PCR, Southern blot or sequencing. Cells with the correct homologous recombination event disrupting the target gene can be tested for target protein expression such as by using ELISA or Western blot analysis and/or mRNA expression such as by RT-PCR. If desired, the selectable marker can be removed by treating the stem cells with Cre recombinase. After Cre recombinase treatment the cells are analyzed for the presence of the nucleic acid encoding the target protein.

Selected stem cells with the correct genomic event disrupting the target gene or introducing the desired nucleic acid sequence can be injected into preimplantation embryos. For microinjection, ES or iPS cell are rendered to single cells using a mixture of trypsin and EDTA, followed by resuspension in ES media. Groups of single cells are selected using a finely drawn-out glass needle (20-25 micrometer inside diameter) and introduced through the embryo's zona pellucida and into the blastocysts cavity (blastocoel) using an inverted microscope fitted with micromanipulators. Alternatively to blastocyst injection, stem cells can be injected into early stage embryos (e.g. 2-cell, 4-cell, 8-cell, premorula or morula). Injection may be assisted with a laser or piezo pulses drilled opening the zona pellucida. Approximately 9-10 selected stem cells (ES or iPS cells) are injected per blastocysts, or 8-cell stage embryo, 6-9 stem cells per 4-cell stage embryo, and about 6 stem cells per 2-cell stage embryo. Following stem cell introduction, embryos are allowed to recover for a few hours at 37°C in 5% CO₂, 5% O₂ in nitrogen or cultured overnight before transfer into pseudopregnant recipient females. In a further alternative to stem cell injection, stem cells can be aggregated with morula stage embryos. All these methods are well established and can be used to produce stem cell chimeras. For a more detailed description see Manipulating the Mouse Embryo: A Laboratory Manual, 3rd edition (A. Nagy, M. Gertsenstein, K. Vintersten, R. Behringer, Cold Spring Harbor Laboratory Press; December 15, 2002, ISBN-10: 0879695919, Nagy et al., 1990, Development 110, 815-821; US7576259: Method for making genetic modifications, US7659442, US 7,294,754, Kraus et al. 2010, Genesis 48, 394-399).

Pseudopregnant embryo recipients are prepared using methods known in the art. Briefly, fertile female mice between 6-8 weeks of age are mated with vasectomized or sterile males to induce a hormonal state conductive to supporting surgically introduced embryos. At 2.5 days post coitum (dpc) up to 15 of the stem cell containing blastocysts are introduced into the uterine horn very near to the uterus-oviduct junction. For early stage embryos and morula, such embryos are either cultured in vitro into blastocysts or implanted into 0.5 dpc or 1.5 dpc pseudopregnant females according to the embryo stage into the oviduct. Chimeric pups from the implanted embryos are born 16-20 days after the transfer depending on the embryo age at implantation. Chimeric males are selected for breeding. Offspring can be analyzed for transmission of the ES cell genome by coat color and nucleic acid analysis, such as PCR, Southern blot or sequencing. Further, the expression of the target gene can be analyzed for target mRNA or protein expression such as by protein analysis, e.g. immunoassay, or functional assays, to confirm target gene disruption. Offspring having the target gene disruption or introduction of a desired nucleic acid sequence are intercrossed to create non-human animals homozygous for the target gene disruption or presence of the desired nucleic acid sequence. The transgenic mice are crossed to the immunodeficient mice to create a congenic immunodeficient strain with the target gene disruption or presence of the desired nucleic acid sequence.

Methods of assessing a genetically modified mouse to determine whether the target gene is disrupted such that the mouse lacks the capacity to express the target gene or whether the desired nucleic acid sequence has been introduced such that the mouse expresses the desired encoded protein are well-known and include standard techniques such as nucleic acid assays, spectrometric assays, immunoassays and functional assays.

One or more standards can be used to allow quantitative determination of target protein in a sample.

Assays for assessment of functional target protein in an animal having a putative disruption of the target gene can be performed. Assays for assessment of function of the target protein in an animal having a putative disruption of the target gene are known in the art as exemplified in Deering et al., Clin Vaccine Immunol January 2006, vol. 13, No. 1, 68-76.

The term "wild-type" refers to a naturally occurring or unmutated organism, protein or nucleic acid.

Optionally, a genetically modified immunodeficient mouse according to the first aspect of the present invention is produced by selective breeding. A first parental strain of non-human animal which has a first desired genotype may be bred with a second parental strain of non-human animal which has a second desired genotype to produce offspring which are genetically modified non-human animals having the first and second desired genotypes. For example, a first mouse which is immunodeficient may be bred with a second mouse which has a Lyst gene disruption such that expression of Lyst is absent or reduced to produce offspring which are immunodeficient and have a Lyst gene disruption such that expression of Lyst is absent or reduced. In further examples, a NOD.Cg-*Prkdc^{scid} Il2rg^{tm1Wjl}*/SzJ mouse, a NOD.Cg-*Rag1^{tm1Mom} Il2rg^{tm1Wjl}l*Szj mouse or a NOD.Cg-*Prkdc^{scid} Il2rg^{tm1Sug}*/JicTac mouse may be bred with a mouse which has a target gene disruption such that expression of the target gene is absent or reduced to produce offspring which are immunodeficient and have a target gene disruption such that expression of the target protein is absent or reduced. In still further examples, a NOD.Cg*-Prkdc^{scid} Il2rg^{tm1Wjl}*/SzJ mouse, a NOD.Cg-*Rag1^{tm1Mom} Il2rg^{tm1Wjl}*/SzJ mouse or a NOD.Cg-*Prkdc^{scid} Il2rg^{tm1Sug}*/JicTac mouse may be bred with a mouse which has an introduced nucleic acid in the genome encoding a protein to be expressed producing offspring which are immunodeficient and express the desired protein encoded by the introduced nucleic acid in the genome.

Otherwise described herein is a genetically modified mouse that includes a target gene disruption in substantially all of their cells, as well as a genetically modified mouse that include a target gene disruption in some, but not all their cells.

Otherwise described herein is a genetically modified immunodeficient mouse that includes a nucleotide sequence encoding a desired protein, such as human CD47 or herpes simplex virus 1 thymidine kinase in substantially all of their cells, as well as a genetically modified immunodeficient mouse that includes a nucleotide sequence encoding a desired protein, such as human CD47 or herpes simplex virus 1 thymidine kinase in some, but not all of their cells. One or multiple copies (such as concatamers) of the nucleotide sequence encoding a desired protein, such as human CD47 or herpes simplex virus 1 thymidine kinase can be integrated into the genomes of the cells of the immunodeficient mouse.

### Mouse Model Including Human Red Blood Cells

A genetically modified immunodeficient mouse may further include human red blood cells.

Human red blood cells can be administered into non-human animals via various routes, such as, but not limited to, intravenous or intraperitoneal administration.

The human red blood cells can be administered one or more times to the genetically modified immunodeficient mouse. Increased survival of human red blood cells in a genetically modified immunodeficient mouse can allow for reduced number of injections of human red bloods cells to allow for assessment of human red blood cells, such as by assays described herein.

Human red blood cells are optionally washed to remove other blood components prior to administration to a genetically modified immunodeficient Lyst^{null} mouse of the first aspect of the present invention. Washing of human red blood cells is accomplished according to various well-known methods, such as by gentle centrifugation of a human whole blood sample to pellet the human red blood cells, removal of the buffy coat and plasma, and resuspension of the human red blood cells in a suitable liquid, such as an iso-osmolar buffer. Optionally, the volume of liquid used to resuspend the washed human red blood cells is substantially equivalent (± 5%) to the original volume of the human whole blood sample so that the washed human red blood cells are termed "undiluted washed human red blood cells."

Otherwise described herein, human red blood cells are introduced to an immunodeficient genetically modified mouse by administration of human hematopoietic stem cells (HSC) which engraft in the immunodeficient mouse and produce human red blood cells by differentiation of the HSC.

The terms "human stem cells" and "human HSC" are used herein refers to multipotent stem cells expressing c-Kit receptor. Examples of multipotent stem cells expressing c-Kit receptor include, but are not limited to, haematopoietic stem cells, also known as hemocytoblasts. C-Kit receptor is well-known in the art, for example as described in Vandenbark GR et al., 1992, Cloning and structural analysis of the human c-kit gene, Oncogene 7(7): 1259-66; and Edling CE, Hallberg B, 2007, c-Kit--a hematopoietic cell essential receptor tyrosine kinase, Int. J. Biochem. Cell Biol. 39(11):1995-8.

Isolation of human HSC, administration of the human HSC to a host mouse and methods for assessing engraftment in the host mouse thereof are well-known in the art.

Human HSC for administration to an immunodeficient mouse can be obtained from any tissue containing human HSC such as, but not limited to, umbilical cord blood, bone marrow, GM-CSF-mobilized peripheral blood and fetal liver.

Human HSC can be administered into newborn mice by administration via various routes, such as, but not limited to, into the heart, liver and/or facial vein. Human HSC can be administered into adult mice by various routes, such as, but not limited to, administration into the tail vein, into the femur bone marrow cavity or into the spleen. In a further example, fetal liver containing the human HSC can be engrafted under the renal capsule.

Administering human HSC to a mouse can include administering a composition comprising human HSC to the mouse. The composition can further include, for example, water, a tonicity-adjusting agent (e.g., a salt such as sodium chloride), a pH buffer (e.g., citrate), and/or a sugar (e.g., glucose).

Engraftment of human HSC in an immunodeficient mouse is characterized by the presence of differentiated human hematopoietic cells in the immunodeficient mice. Engraftment of human HSC can be assessed by any of various methods, such as, but not limited to, flow cytometric analysis of cells in the animals to which the human HSC are administered at one or more time points following the administration of human HSC.

Exemplary methods for isolation of human HSC, administration of the human HSC to a host mouse and methods for assessing engraftment thereof are described herein and in T. Pearson et al., Curr. Protoc. Immunol. 81:15.21.1-15.21.21, 2008; Ito, M. et al, Blood 100: 3175-3182; Traggiai, E. et al, Science 304: 104-107; Ishikawa, F. et al, Blood 106: 1565-1573; Shultz, L. D. et al, J. Immunol. 174: 6477-6489; Holyoake TL et al, Exp Hematol., 1999, 27(9):1418-27.

Otherwise described herein, the human HSC administered to an immunodeficient mouse are isolated from an original source material to obtain a population of cells enriched in human HSC. The isolated human HSC may or may not be pure. According to aspects, human HSC are purified by selection for a cell marker, such as CD34. According to aspects, administered human HSC are a population of cells in which CD34+ cells constitute about 1- 100% of total cells, although a population of cells in which CD34+ cells constitute fewer than 1% of total cells can also be used. According to embodiments, administered human HSC are T cell depleted cord blood cells in which CD34+ cells make up about 1-3% of total cells, lineage depleted cord blood cells in which CD34+ cells make up about 50% of total cells, or CD34+ positively selected cells in which CD34+ cells make up about 90% of total cells.

The number of HSCs administered is not considered limiting with regard to generation of human red blood cells in an immunodeficient genetically modified mouse described herein. A single HSC can generate red blood cells in a host immunodeficient genetically modified mouse. Thus, the number of administered HSCs is generally in the range of 1×10³ to 1×10⁶ (1,000 to 1,000,000) CD34+ cells where the recipient is a mouse, although more or fewer can be used.

Otherwise described herein is a method including administering about 10³ (1000) to about 10⁶ (1,000,000), about 10³ (1000) to about 10⁵ (100,000), about 10⁴ (10,000) to about 10⁶ (1,000,000), about 10⁵ (100,000) to about 10⁷ (10,000,000), about 1 × 10³ (1,000) to about 1 × 10⁴ (10,000), about 5 × 10³ (5,000) to about 5 × 10⁴ (50,000), about 1 × 10⁴ (10,000) to about 1 × 10⁵ (100,000), about 5 × 10⁴ (50,000), to about 5 x 10⁵ (500,000), about 1 × 10⁵ (100,000) to about 1 × 10⁶ (1,000,000), about 5 × 10⁵ (500,000) to about 5 × 10⁶ (5,000,000), about 1 × 10⁶ (1,000,000), to about 1 × 10⁷ (10,000,000), about 2 × 10⁴ (20,000) to about 5 × 10⁵ (500,000), or about 5 × 10⁴ (50,000) to about 2 × 10⁵ (200,000), human HSC to the immunodeficient genetically modified mouse. The method can include administering at least about 1 × 10², about 2 x 10², about 3 × 10², about 4 × 10², about 5 × 10², about 6 × 10², about 7 × 10², about 8 × 10², about 9 × 10², about 1 × 10³, about 2 × 10³, about 3 × 10³, about 4 × 10³, about 5 × 10³, about 6 × 10³, about 7 × 10³, about 8 × 10³, about 9 × 10³, about 1 × 10⁴, about 2 × 10⁴, about 3 × 10⁴, about 4 × 10⁴, about 5 × 10⁴, about 6 × 10⁴, about 7 × 10⁴, about 8 × 10⁴, about 9 × 10⁴, about 1 × 10⁵, about 2 × 10⁵, about 3 × 10⁵, about 4 × 10⁵, about 5 × 10⁵, about 6 × 10⁵, about 7 × 10⁵, about 8 × 10⁵, about 9 × 10⁵, about 1 × 10⁶, about 2 × 10⁶, about 3 × 10⁶, about 4 × 10⁶, about 5 × 10⁶, about 6 × 10⁶, about 7 × 10⁶, about 8 × 10⁶, about 9 × 10⁶, or about 1 × 10⁷ human HSC, to the immunodeficient genetically modified mouse. Those of ordinary skill will be able to determine a number of human HSC to be administered to a specific mouse using no more than routine experimentation.

Engraftment is successful where human HSCs and/or cells differentiated from the human HSCs in the recipient immunodeficient genetically modified mouse are detected at a time when the majority of any administered non-HSC have degenerated. Detection of differentiated HSC can be achieved by detection of human red blood cells in a sample obtained from the mouse following administration of the human HSC.

Engraftment of human HSC in an immunodeficient genetically modified mouse may include "conditioning" of the immunodeficient genetically modified mouse prior to administration of the human HSC, for example by sub-lethal irradiation of the recipient animal with high frequency electromagnetic radiation, generally using gamma radiation, or treatment with a radiomimetic drug such as busulfan or nitrogen mustard. Conditioning is believed to reduce numbers of host hematopoietic cells, create appropriate microenvironmental factors for engraftment of human HSC, and/or create microenvironmental niches for engraftment of human HSC. Standard methods for conditioning are known in the art, such as described herein and in J. Hayakawa et al, 2009, Stem Cells, 27(1):175-182.

Otherwise described herein are methods which include administration of human HSC to an immunodeficient genetically modified mouse without "conditioning" the immunodeficient genetically modified mouse prior to administration of the human HSC. Methods may include administration of human HSC to an immunodeficient genetically modified mouse without "conditioning" by radiation or radiomimetic drugs of the immunodeficient genetically modified mouse prior to administration of the human HSC.

### Infection

According to the first aspects, human red blood cells administered to a genetically modified immunodeficient Lyst^{null} mouse of the present invention are infected with an infectious agent.

According to the first aspect, human red blood cells administered to a genetically modified immunodeficient Lyst^{null} mouse of the present invention are infected with a *Plasmodium* parasite. The *Plasmodium* parasite is *Plasmodium falciparum (P. falciparum)* according to the first aspect of the present invention. The *Plasmodium* parasite is *Plasmodium ovale (P. ovale,)*, *Plasmodium vivax (P. vivax,)*, *or Plasmodium malariae (P. malariae,)* according to the first aspect of the present invention.

Otherwise described herein is an infectious agent administered to a genetically modified immunodeficient mouse, wherein the genetically modified immunodeficient mouse includes human RBC and the infectious agent is capable of infecting the human RBC. The infectious agent administered to the genetically modified immunodeficient mouse may be a *Plasmodium* parasite. The *Plasmodium* parasite may be *Plasmodium falciparum (P. falciparum).* The *Plasmodium* parasite may be *Plasmodium ovale (P. ovale), Plasmodium vivax (P. vivax)*, or *Plasmodium malariae (P. malariae).*

### Disease

Human red blood cells administered to a genetically modified immunodeficient mouse could be affected by a disorder or disease.

Human red blood cells administered to a genetically modified immunodeficient mouse could be derived from an individual human or population of human individuals (e.g. pooled samples) wherein the individual human or population of human individuals could have sickle cell anemia.

### Assays

Methods of assaying an effect of a putative therapeutic agent are provided according to the second aspect of the present invention which include administering an amount of the putative therapeutic agent to a genetically modified immunodeficient mouse including human red blood cells; and measuring the effect of the putative therapeutic agent.

A putative therapeutic agent used in a method of the present invention can be any chemical entity, illustratively including a synthetic or naturally occurring compound or a combination of a synthetic or naturally occurring compound, a small organic or inorganic molecule, a protein, a peptide, a nucleic acid, a carbohydrate, an oligosaccharide, a lipid or a combination of any of these.

Methods of assaying an effect of a putative therapeutic agent are provided according to the second aspect of the present invention and may include: treating a mouse with a macrophage toxin producing a treated mouse with fewer than normal macrophages; administering human red blood cells to the treated mouse; administering an amount of the putative therapeutic agent to the treated mouse; and measuring the effect of the putative therapeutic agent. Non-limiting examples of macrophage toxins include bisphosphonates, such as but not limited to zoledronate, clodronate, pamidronate and ibandronate.

Methods of assaying an effect of a putative therapeutic agent are provided according to the second aspect of the present invention and may include: treating a genetically modified immunodeficient mouse, wherein the genetically modified immunodeficient mouse is a genetically modified immunodeficient mouse according to the second aspect of the present invention, with a macrophage toxin producing a treated genetically modified immunodeficient mouse with fewer than normal macrophages; administering human red blood cells to the treated genetically modified immunodeficient mouse; administering an amount of the putative therapeutic agent to the treated genetically modified immunodeficient mouse; and measuring the effect of the putative therapeutic agent. Non-limiting examples of macrophage toxins include bisphosphonates, such as but not limited to zoledronate, clodronate, pamidronate and ibandronate.

Methods of assaying an effect of a putative therapeutic treatment are provided according to the second aspect of the present invention and may include administering a putative therapeutic treatment to a genetically modified immunodeficient mouse including human red blood cells; and measuring the effect of the putative therapeutic treatment. As a non-limiting example, the genetically modified immunodeficient mouse can be further genetically modified, such as by gene therapy to treat a disorder or disease of red blood cells. Disorders and diseases of red blood cells include, but are not limited to, malaria and other infectious diseases of red blood cells, sickle cell anemia, and the like.

Therapeutic agents for treatment of malaria can be administered to a genetically modified immunodeficient mouse including human red blood cells, wherein the human red blood cells may be infected with a *Plasmodium* parasite such as *P. falciparum, P. ovale, P. vivax, or P. malariae.* Examples of therapeutic agents for treatment of malaria include amodiaquine; artemisinin; artemisinin derivatives, such as artesunate, artemether, dihydroartemisinin, artelinic acid, and artemotil; atovaquone; chloroguanide (proguanil); chloroquine; cinchoine; cinchonidine; clindamycin; doxycycline; halofantrine, hydroxychloroquine; lumefantrine; mefloquine; piperaquine; primaquine, pyrimethamine; pyronaridine; quinine; quinidine; sulfadoxine; tafenoquine; and tetracycline.

Otherwise described herein are one or more therapeutic agents incorporated into human red blood cells and administered to an immunodeficient genetically modified mouse to determine the effect of the therapeutic agent. A therapeutic agent can be incorporated into human RBC, for example, by incubation of the therapeutic agent and the RBC together to allow diffusion or active uptake of the therapeutic agent into the RBC or by administration, such as by microinjection. The therapeutic agent may be associated with a carrier which stimulates uptake by RBC, such as, but not limited to, liposomes.

Standards suitable for assays are well-known in the art and the standard used can be any appropriate standard.

Assay results can be analyzed using statistical analysis by any of various methods, exemplified by parametric or non-parametric tests, analysis of variance, analysis of covariance, logistic regression for multivariate analysis, Fisher's exact test, the chi-square test, Student's T-test, the Mann-Whitney test, Wilcoxon signed ranks test, McNemar test, Friedman test and Page's L trend test. These and other statistical tests are well-known in the art as detailed in Hicks, CM, Research Methods for Clinical Therapists: Applied Project Design and Analysis, Churchill Livingstone (publisher); 5th Ed., 2009; and Freund, RJ et al., Statistical Methods, Academic Press; 3rd Ed., 2010.

Embodiments of inventive compositions and methods are illustrated in the following examples. These examples are provided for illustrative purposes and are not considered limitations on the scope of inventive compositions and methods. To the extent that any of the following examples fall outside the scope of the accompanying claims, they are included as comparative examples only.

### Examples

### Methods and Materials

### Animals

Mice used *in* this study were on the NSG or C57BL/6 strain background and raised at The Jackson *Laboratory* (Bar Harbor, ME). Male and female mice aged from two to four months were used. All animals were housed in a BSL2 certified room as injection of human blood was a main component of the project. Standard bedding, food and water were available ad libitum. Cages were maintained at 23°C on a 12h-light/dark cycle (lights on at 6:00). NSG mice represent a "standard" strain and were therefore used as the control against other newly developed strains.

### Human blood samples

Human blood samples in heparin were received weekly. Once obtained, blood was tested for Lymphocytic *Choriomeningitis* Virus (LCMV) and bacteria before use.

### Testing Procedures

Each separate experiment consisted of 5 NSG mice and 5 next generation NSG mice or BL/6 Rag gamma CD47 (MD4) KO mice. All experiments were conducted in a designated BSL2 certified room. 200ul of washed undiluted human red blood cells was injected into the mouse by either intravenous or intraperitoneal injections. The type of injection was kept consistent throughout each respective experiment. After each initial injection, mice were bled from the tail at predetermined time points.

### Administration of Ganciclovir

Ganciclovir (GVC) was administered to the NSG MD3 mouse strain in order to induce ablation of macrophages. GVC was administered to five mice for four consecutive days prior to the beginning of the experiment.

### Antibody cocktails

Two different antibody mixtures were used: 1) FITC Glycophorin A (GPA) mixed with APC Ter-119 and FACS buffer and 2) FITC CD41b mixed with APC Ter-119, PE Glycophorin A and FACS buffer. To make the former, the FITC GPA was used at a 1:500 dilution while the APC Ter-119 was used at a 1:50 dilution. The latter was made using FITC CD41B at a 1:20 dilution, APC Ter-119 at a 1:50 dilution and PE GPA at a 1:500 dilution. All mixtures were vortexed to ensure thorough mixing.

### Flow Cytometry

In *order* to quantitate and differentiate cell populations from peripheral blood using Fluorescein isothiocyanate (FITC) For Human Glycophorin A (GPA) at a 1:500 dilution (E-Biosciences) and Allophycocyanin (APC) at a 1:50 dilution for each sample, with 50ul of antibody cocktail per 2ul of blood. The antibody was mixed with the blood and refrigerated for 30-60 minutes at 4°C to ensure sufficient staining. The blood was then resuspended in ImL of PBS Azide. Flow cytometry was performed using an Attune flow cytometer (Thermo Fisher Scientific, Waltham, MA) as per the manufacturer's protocol. Samples were then quantified using the cell analyzing software FlowJo (FlowJo LLC, Ashland, OR).

### Results

### Genetically Engineering a Mouse for improved human RBC survival

### NSG Lyst (MD1) KO mouse strain (NSG MD1 mice)

This mouse is unique in that it has had the Lyst gene knocked out, a gene that when mutated/absent can result in a human disease syndrome. Without this gene, the mouse's innate immune system is compromised resulting in lysosomal dysfunction and immune cell dysfunction.

Generation of an Immunodeficient Genetically Modified Mouse - NSG Lyst knock out MD1

The NSG Lyst knock out MD1 mouse was generated at The Jackson Laboratory by pronuclear injection of Cas9 RNA (100 ng) and a single guide sequence (50 ng), sgRNA-1537 (ATCCGTTGAACCAAAGCTAC, SEQ ID NO:2) into NSG fertilized oocytes.

For sgRNA-1537: Transferred 55 embryos (3 pseudos), 14 liveborn, 2/14 (14%) NHEJ

The CRISPR strategy resulted in the specific deletion of a 25 base pair deletion (GAGCCGGTAGCTTTGGTTCAACGGA , SEQ ID NO:1) in exon 5 of the mouse Lyst gene.

Four primers were designed for genotyping:
Primer #1578
   GGGTGAATATTGAAGTTCTGAGAC (SEQ ID NO: 3)
Primer #1579
   CATTTGAATCCTGTCTCAGAATGA (SEQ ID NO: 4)
Primer #1580
   GCCACCAAAGAACAGGTCCTTT (SEQ ID NO: 5)
Primer #1581
   GAAGTGGGAATACTCACAACGC (SEQ ID NO: 6)

To genotype sg1537-targeted mutants, use primers 1580/1581 for genotyping PCR and sequencing. The product should be ~903bp, and the optimal PCR program using NEB Standard Taq (M0273) follows:

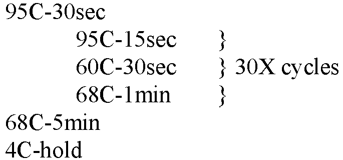

Founder mutants identified from the 1537-sgRNA targeted set are: #7 and #12.

>1580/1581 Amplicon (903bp) (SEQ ID NO: 7)

>1578/1581 Amplicon (1625bp) (SEQ ID NO: 8)

Offspring carrying the modified allele in the germ-line were interbred to generate the homozygous genetically modified genome. All F1 matings produced normal litter sizes with a Mendelian distribution of the locus. The resulting inbred strain of mouse is designated NSG Lyst (MD1) KO mouse strain (NSG MD1 mice) which does not express functional Lyst protein.

Figure 1 shows the retention of human RBC from these NSG Lyst (MD1) knockout (KO) mice compared to NSG control mice. Although the NSG Lyst (MD1) KO appears to retain human RBC more efficiently than the NSG mice, there was not statistical significance beyond 24 hours in this experiment.

Figure 1: NSG vs NSG MD 1. The graph shows RBC survival within NSG (solid line) when compared to NSG MD1 KO (broken line). NSG MD1 mice did retain human at a higher rate than NSG. Data was not significant up to 24 hours in this experiment (P value > 0.1). Intraperitoneal injection.

### NSG mouse strain including transgene in which the CSFlr promoter drives expression of herpes thymidine kinase (NSG-MD3 mice)

Mouse cells do not typically express herpes simplex virus 1 thymidine kinase (HSV-1-tk), a derivative of the herpes virus. However, by attaining a mouse model that expresses cell-specific HSV-1-tk on its macrophages, conditional ablation of macrophages was attained. HSV-1-tk alone is not lethal to mammalian cells.

The gene encoding the receptor for macrophage colony-stimulating factor (CSF-1R) is expressed exclusively in cells of the myeloid lineages as well as trophoblasts. A conserved element in the second intron, Fms-Intronic Regulatory Element (FIRE), is essential for macrophage-specific transcription of the gene, see for example, Sasmono R T et al. Blood 2003;101:1155-1163

A BAC clone carrying the mouse Csflr Gene: RP23-30G17 was used to clone the mouse Csflr promoter into a vector, then three-way ligation was used to introduce an IRES and HSV TK Gene/pA provided as PCR Amplicons to produce an expression construct in which HSV TK is driven by Csfrlr in pUC57 simple. The total size of this transgene is 9,850 bp including the 7,510 bp of the mouse Csflr 5' flanking region and sequences extending into 5' end of exon 3. The HSV TK gene is expressed 3' to the IRES element and HSV TK gene transcription is terminated by the TK pA sequence

For generation of these mice, the expression construct encoding HSV-1-tk under control of the CSFlr (colony stimulating factor 1 receptor) promoter is injected into NOD x NOD scid fertilized eggs. A transgenic founder is then crossed with NSG to establish the NSG HSV-1-tk Tg strain. To make the NSG strain homozygous for the HSV-1-tk transgene, the two strains are intercrossed.

The cell-specific expression of herpes simplex virus 1 thymidine kinase (HSV-1-tk) provides a simple and highly efficient technique to achieve conditional ablation of targeted cell types in transgenic mice. The ablation is induced by treating transgenic animals expressing HSV-1-tk with the antiherpetic drug ganciclovir. In tissues of mice expressing HSV-1-tk driven by the Csflr promoter, administration of ganciclovir is expected to lead to destruction of cells within the macrophage lineage. Tissues not expressing HSV-1-tk are insensitive to drug treatment.

Ganciclovir is a synthetic analogue of 2'-deoxy-guanosine. It is first phosphorylated to ganciclovir monophosphate by viral kinases. Subsequently, cellular kinases catalyze the formation of ganciclovir diphosphate and ganciclovir triphosphate, which is present in 10-fold greater concentrations in CMV or herpes simplex virus (HSV)-infected cells than uninfected cells. Ganciclovir triphosphate is a competitive inhibitor of deoxyguanosine triphosphate (dGTP) incorporation into DNA and preferentially inhibits viral DNA polymerases more than cellular DNA polymerases. In addition, ganciclovir triphosphate serves as a poor substrate for chain elongation, thereby disrupting viral DNA synthesis by a second route. The macrophages within the transgenic mice (NSG-Tg(Csflr-HSV-1-tk) will be killed by the gancyclovir similarly to a Herpes virus infected cell.

HSV-1-k is however able to phosphorylate specific nucleoside analogs. These nucleoside monophosphates phosphorylated by cellular kinases to the nucleoside triphosphate and incorporated into DNA which in turn leads to cells death. In order to induce macrophage death, NSG-MD3 mice were treated with the antiherpetic gancyclovir for four consecutive days prior to experimentation. Figure 2 depicts the results from these HSV-1-tk Tg mice when compared to NSG control mice. Again, the number of human RBCs that survived in the NSG MD3 mice was higher than in NSG controls at the time points measured. When the raw data was put through a paired t-test, there was a slight significance difference (P< 0.05) of up to 24 hours.

Figure 2: NSG vs NSG MD3. The graph shows RBC survival within NSG (solid line) when compared to NSG MD3 (broken line). NSG MD3 mice did retain human at a higher rate than NSG. Data was significant up to 24 hours (P value < 0.05). Intraperitoneal injection.

B6.129S-Rag1<tm1Mom> CD47 KO Il2rg<tm1Wjl>/Sz (BL/6 Rag gamma MD4).

Among all the mice generated, this was the only mouse strain of the BL/6 background. In addition, this mouse has CD47 knocked out of its genome. Mouse RBCs express CD47 that is ubiquitously expressed throughout the body. CD47 interacts with an inhibitory immunoreceptor, an engagement that administers a regulatory signal which in turn inhibits cell phagocytosis. Essentially, this protein functions as a "don't-eat-me" signal. Although human RBC express CD47, it is not homologous with murine CD47. This lack of homology is what predominantly causes recognition of the human RBC as foreign and subsequent clearance. Figure 3 depicts these BL/6 Rag gamma MD4 mice when compared to NSG control mice.

Interestingly, human RBC were cleared more rapidly within these BL/6 Rag gamma MD4 mice than in NSG controls.

Figure 3: NSG vs BL/6 Rag gamma MD4. The graph shows RBC survival within NSG (solid line) when compared to BL/6 rag gamma MD4 (broken line). BL/6 Rag Gamma MD4 KO mice did not retain human at a higher rate than NSG. Data was significant (P>0.01). Intraperitoneal injection.

NOD.Cg-Prkdc<scid> CD47 KO Il2rg<tm1Wjl> Tg(hMD2)Sz/Sz (NSG MD2).

Similar to the BL/6 Rag gamma MD4 mice, NSG MD2 mice have had the gene that encodes for the murine version of CD47 knocked out. In addition to this genetic modification, the human version of CD47 has been knocked into their respective genome.

For generation of these mice, a CD47 knockout allele (see, e.g. Oldenborg et al., Lethal autoimmune hemolytic anemia in CD47-deficient nonobese diabetic (NOD) mice, Blood, 2002 May 15;99(10):3500-4) was backcrossed onto the NSG background to generate NSG CD47 Knockout mice. A bacterial artificial chromosome, BAC RP11-121A9, encoding human CD47 was injected into NOD x NOD scid fertilized eggs. One transgenic out of 32 potential founders was present. This founder was crossed with NSG to establish the NSG human CD47 Tg strain. To make the NSG strain homozygous for the mouse CD47 KO and the human CD47 transgene, the two strains were intercrossed and fixed all of the CD47 alleles to homozygosity.

Figure 4 depicts these NSG MD2 mice when compared to NSG control mice.

In multiple experiments with the NSG MD2 strain of mouse, significant data was obtained, transgenically introducing human CD47 into the NSG mouse effectively improved RBC survival. As can be in Figure 4, while human RBC survival in NSG mice only lasted just over 40 hours, human RBC survival in the NSG MD2 mice lasted nearly 100 hours (P value: 0.009).

Figure 4: NSG vs NSG MD2. The graph shows RBC survival within NSG (solid line) when compared to NSG MD2 mice (broken line). NSG MD2. mice did retain human at a higher rate than NSG. Data was significant (P value < 0.001). Intraperitoneal injection.

Figure 5 shows results comparing human RBC survival in several different genetically modified immunodeficient strains. As can been seen, the data demonstrate human RBCs circulating within NSG MD1 and MD2 mice up to 96 hours.

Figure 5: NSG vs NSG MD1 vs NSG MD2 vs BL/6 Rag Gamma MD4. The comparison shows multiple strains tested at once. Intraperitoneal injection.

The compositions and methods described herein are presently representative of preferred embodiments, exemplary, and not intended as limitations on the scope of the invention. Changes therein and other uses will occur to those skilled in the art. Such changes and other uses can be made without departing from the scope of the invention as set forth in the claims.

## Claims

1. A genetically modified immunodeficient mouse whose genome comprises a genetic modification, wherein the genetic modification renders the mouse deficient in macrophages and/or macrophage anti-human red blood cell activity so as to prolong the survival of human red blood cells when administered into said mouse, wherein the genetic modification is a mutation of a lysosomal trafficking regulator gene, wherein the mouse is a Lyst^{null} immunodeficient mouse.

2. The genetically modified immunodeficient mouse of claim 1, wherein the mouse is an NRG, NSG or NOG mouse.

3. The genetically modified immunodeficient mouse of claim 1or 2, wherein the mouse is a NOD.Cg-*Prkdc^{scid} Il2rg^{tm1Wjl}*/Lyst <emlMvw>/Sz (NSG Lyst knock out) mouse.

4. The genetically modified immunodeficient mouse of any of claims 1 to 3, further comprising human red blood cells in the blood system of the non-human animal or further comprising engrafted human hematopoietic cells.

5. The genetically modified immunodeficient mouse of any of claims 1 to 4, wherein human red blood cells survive longer in the mouse than in an immunodeficient mouse of the same type whose genome does not include the genetic modification, and/or wherein the human red blood cells are derived from an individual human or population of human individuals, wherein the individual human or population of human individuals have sickle cell anemia.

6. The genetically modified immunodeficient mouse of any of claims 1 to 5, wherein human red blood cells are infected by an infectious agent, and/or the mouse has been administered an infectious agent capable of infecting human red blood cells.

7. The genetically modified immunodeficient mouse of claim 6, wherein the infectious agent is a *Plasmodium* parasite.

8. The genetically modified immunodeficient mouse of claim 7, wherein the infectious agent is *Plasmodium falciparum (P. falciparum).*

9. The genetically modified immunodeficient mouse of claim 8 wherein the infectious agent is *Plasmodium ovale (P. ovale,)*, *Plasmodium vivax (P. vivax,)*, *or Plasmodium malariae (P. malariae).*

10. A method of assaying an effect of a putative therapeutic agent, comprising:
administering an amount of the putative therapeutic agent to a genetically modified immunodeficient mouse comprising human red blood cells of any one of claims 4 to 9; and measuring the effect of the putative therapeutic agent.

## Patentansprüche

1. Genetisch modifizierte immundefiziente Maus, deren Genom eine genetische Modifikation umfasst, wobei die genetische Modifikation der Maus ein Defizit an Makrophagen und/oder Makrophagen-Aktivität von Anti-Mensch-rote-Blutkörperchen verleiht, um das Überleben menschlicher roter Blutkörperchen zu verlängern, wenn sie der Maus verabreicht werden, wobei die genetische Modifikation eine Mutation eines lysosomalen Trafficking-Regulatorgens ist, wobei die Maus eine immundefiziente Lyst^{null}-Maus ist.

2. Genetisch modifizierte immundefiziente Maus nach Anspruch 1, wobei die Maus eine NRG-, NSG- oder NOG-Maus ist.

3. Genetisch modifizierte immundefiziente Maus nach Anspruch 1 oder 2, wobei die Maus eine *NOD.Cg-Prkdc^{scid}-ll2rg^{tmlWjl}*/*Lyst*<em1Mvw>/Sz(NSG Lyst knock out)-Maus ist.

4. Genetisch modifizierte immundefiziente Maus nach einem der Ansprüche 1 bis 3, die ferner menschliche rote Blutkörperchen in dem Blutsystem des nichtmenschlichen Tieres umfasst oder ferner transplantierte menschliche blutbildende Zellen umfasst.

5. Genetisch modifizierte immundefiziente Maus nach einem der Ansprüche 1 bis 4, wobei menschliche rote Blutkörperchen in der Maus länger überleben als in einer immundefizienten Maus derselben Art, deren Genom die genetische Modifikation nicht beinhaltet, und/oder wobei die menschlichen roten Blutkörperchen von einem individuellen Menschen oder einer Population menschlicher Individuen abstammen, wobei der individuelle Mensch oder die Population menschlicher Individuen Sichelzellenanämie aufweist.

6. Genetisch modifizierte immundefiziente Maus nach einem der Ansprüche 1 bis 5, wobei menschliche rote Blutkörperchen durch ein infektiöses Mittel infiziert sind und/oder der Maus ein infektiöses Mittel verabreicht worden ist, das in der Lage ist, menschliche rote Blutkörperchen zu infizieren.

7. Genetisch modifizierte immundefiziente Maus nach Anspruch 6, wobei das infektiöse Mittel ein *Plasmodium-Parasit* ist.

8. Genetisch modifizierte immundefiziente Maus nach Anspruch 7, wobei das infektiöse Mittel *Plasmodium falciparum (P. falciparum) ist.*

9. Genetisch modifizierte immundefiziente Maus nach Anspruch 8, wobei das infektiöse Mittel *Plasmodium ovale (P. ovale), Plasmodium vivax (P. vivax) oder Plasmodium malariae* (*P. malariae) ist.*

10. Verfahren zum Untersuchen einer Wirkung eines vermeintlichen therapeutischen Mittels, das Folgendes umfasst:
Verabreichen einer Menge des vermeintlichen therapeutischen Mittels an eine genetisch modifizierte immundefiziente Maus, die menschliche rote Blutkörperchen nach einem der Ansprüche 4 bis 9 umfasst; und Messen der Wirkung des vermeintlichen therapeutischen Mittels.

## Revendications

1. Souris immunodéficiente génétiquement modifiée dont le génome comprend une modification génétique, dans laquelle la modification génétique rend la souris déficiente en macrophages et/ou en activité macrophage anti-globules rouges humains de manière à prolonger la survie des globules rouges humains lorsqu'ils sont administrés à ladite souris, la modification génétique étant une mutation d'un gène régulateur de trafic lysosomal, la souris étant une souris immunodéficiente Lyst^{nulle}.

2. Souris immunodéficiente génétiquement modifiée selon la revendication 1, dans laquelle la souris est une souris NRG, NSG ou NOG.

3. Souris immunodéficiente génétiquement modifiée selon la revendication 1 ou 2, dans laquelle la souris est une souris *NOD.Cg-Prkdc^{scid} ll2rg^{tmlWjl}*/*Lyst* <em1Mvw>/Sz (invalidation génétique NSG Lyst).

4. Souris immunodéficiente génétiquement modifiée selon l'une quelconque des revendications 1 à 3, comprenant en outre des globules rouges humains dans le système sanguin de l'animal non humain ou comprenant en outre des cellules hématopoïétiques humaines greffées.

5. Souris immunodéficiente génétiquement modifiée selon l'une quelconque des revendications 1 à 4, dans laquelle les globules rouges humains survivent plus longtemps dans la souris que dans une souris immunodéficiente du même type dont le génome ne comprend pas la modification génétique, et/ou dans laquelle les globules rouges humains sont dérivés d'un être humain individuel ou d'une population d'individus humains, l'être humain individuel ou la population d'individus humains étant atteint d'anémie falciforme.

6. Souris immunodéficiente génétiquement modifiée selon l'une quelconque des revendications 1 à 5, dans laquelle les globules rouges humains sont infectés par un agent infectieux, et/ou la souris a reçu un agent infectieux capable d'infecter les globules rouges humains.

7. Souris immunodéficiente génétiquement modifiée selon la revendication 6, dans laquelle l'agent infectieux est un parasite *Plasmodium.*

8. Souris immunodéficiente génétiquement modifiée selon la revendication 7, dans laquelle l'agent infectieux est *Plasmodium falciparum (P. falciparum).*

9. Souris immunodéficiente génétiquement modifiée selon la revendication 8, dans laquelle l'agent infectieux est *Plasmodium ovale (P. ovale,), Plasmodium vivax (P. vivax,) ou Plasmodium malariae (P. malariae).*

10. Procédé de dosage d'un effet d'un agent thérapeutique putatif, comprenant :
l'administration d'une quantité de l'agent thérapeutique putatif à une souris immunodéficiente génétiquement modifiée comprenant des globules rouges humains selon l'une quelconque des revendications 4 à 9 ; et la mesure de l'effet de l'agent thérapeutique putatif.


### SEQUENCE LISTING

<110> The Jackson Laboratory
<120> METHODS AND COMPOSITIONS RELATING TO IMPROVED HUMAN
<130> JLA-11852/47
<150> 62/373,671 <151> 2016-08-11
<160> 9
<170> PatentIn version 3.5
<210> 1
   <211> 25
   <212> DNA
   <213> Artificial Sequence
<220>
   <223> sequence deleted from mouse Lyst gene
<400> 1
   gagccggtag ctttggttca acgga 25
<210> 2
   <211> 20
   <212> DNA
   <213> Artificial Sequence
<220>
   <223> single guide RNA for CRISPR-mediated deletion in mouse Lyst gene
<400> 2
   atccgttgaa ccaaagctac 20
<210> 3
   <211> 24
   <212> DNA
   <213> Artificial Sequence
<220>
   <223> PCR primer
<400> 3
   gggtgaatat tgaagttctg agac 24
<210> 4
   <211> 24
   <212> DNA
   <213> Artificial Sequence
<220>
   <223> PCR primer
<400> 4
   catttgaatc ctgtctcaga atga 24
<210> 5
   <211> 22
   <212> DNA
   <213> Artificial Sequence
<220>
   <223> PCR primer
<400> 5
   gccaccaaag aacaggtcct tt 22
<210> 6
   <211> 22
   <212> DNA
   <213> Artificial Sequence
<220>
   <223> PCR primer
<400> 6
   gaagtgggaa tactcacaac gc 22
<210> 7
   <211> 903
   <212> DNA
   <213> Artificial Sequence
<220>
   <223> Mouse Lyst amplicon
<400> 7
   gccaccaaag aacaggtcct ttctgacacc atgtctgtgg aaaactccag agaagtcatt      60
   ctgagacagg attcaaatgg tgacatatta agtgagccag ctgctttgtc tattctcagt     120
   aacatgaata attctccttt tgacttatgt catgttttgt tatctctatt ggaaaaagtt     180
   tgtaagtttg acattgcttt gaatcataat tcttccctag cactcagtgt agtacccaca     240
   ctgactgagt tcctagcagg ctttggggac tgctgtaacc agagtgacac tttggaggga     300
   caactggttt ctgcaggttg gacagaagag ccggtagctt tggttcaacg gatgctcttt     360
   cgaaccgtgc tgcaccttat gtcagtagac gttagcactg cagaggcaat gccagaaagt     420
   cttaggaaaa atttgactga attgcttagg gcagctttaa aaattagagc ttgcttggaa     480
   aagcagcctg agcctttctc cccgagacaa aagaaaacac tacaggaggt ccaggagggc     540
   tttgtatttt ccaagtatcg tcaccgagcc cttctactac ctgagcttct ggaaggagtt     600
   ctacagctcc tcatctcttg tcttcagagt gcagcttcaa atccctttta cttcagtcaa     660
   gccatggatt tagttcaaga atttatccag caccaaggat ttaatctctt tgaaacagca     720
   gttcttcaga tggaatggct gctttcaagg gacggtgttc cttcagaagc tgcagaacat     780
   ttgaaagctc tgataaacag tgtaataaaa ataatgagta ctgtgaaaaa ggtgaaatca     840
   gagcaacttc atcattccat gtgcacaagg aaaagacacc ggcgttgtga gtattcccac     900
   ttc                                                                   903
<210> 8
   <211> 1625
   <212> DNA
   <213> Artificial Sequence
<220>
   <223> Mouse Lyst amplicon
<400> 8
   gggtgaatat tgaagttctg agacactata aactactcta tgtcttaatt ttaacattac 60
   taaagatttc taaatggtga gcacagcaac tggataaccc agagtctcat attttgaaat 120
   cacaatgcaa tatataggtt caacttaggt ctactttcct aactcttcct tgctattttc 180
   aaatcagttt gtatcccctg agctaattcc acttggcatt gagaattaaa aagataagtg 240
   tggggagaag tgacccagag atgcaactag ggaaagcagc cttgaaggga aattatgcca 300
   ggccagactc atgccggttg tgagtcattg cctgtgtgtt taacagttac taacctaaga 360
   cttctttctt gatttcatta gattttaacc tgccactgtc atctgatata atcctgacca 420
   aagaaaagaa ctcaagtttg caaaaatcaa ctcagggaaa attatattta gaaggaagtg 480
   ctccatctgg tcaggtttct gcaaaagtaa acctttttcg aaaaatcagg cgacagcgta 540
   aaagtaccca tcgttattct gtaagagatg caagaaagac acagctctcc acctctgact 600
   ccgaaggcaa ctcagatgaa aagagtacgg ttgtgagtaa acacaggagg ctccacgcgc 660
   tgccacggtt cctgacgcag tctcctaagg aaggccacct cgtagccaaa cctgacccct 720
   ctgccaccaa agaacaggtc ctttctgaca ccatgtctgt ggaaaactcc agagaagtca 780
   ttctgagaca ggattcaaat ggtgacatat taagtgagcc agctgctttg tctattctca 840
   gtaacatgaa taattctcct tttgacttat gtcatgtttt gttatctcta ttggaaaaag 900
   tttgtaagtt tgacattgct ttgaatcata attcttccct agcactcagt gtagtaccca 960
   cactgactga gttcctagca ggctttgggg actgctgtaa ccagagtgac actttggagg 1020
   gacaactggt ttctgcaggt tggacagaag agccggtagc tttggttcaa cggatgctct 1080
   ttcgaaccgt gctgcacctt atgtcagtag acgttagcac tgcagaggca atgccagaaa 1140
   gtcttaggaa aaatttgact gaattgctta gggcagcttt aaaaattaga gcttgcttgg 1200
   aaaagcagcc tgagcctttc tccccgagac aaaagaaaac actacaggag gtccaggagg 1260
   gctttgtatt ttccaagtat cgtcaccgag cccttctact acctgagctt ctggaaggag 1320
   ttctacagct cctcatctct tgtcttcaga gtgcagcttc aaatcccttt tacttcagtc 1380
   aagccatgga tttagttcaa gaatttatcc agcaccaagg atttaatctc tttgaaacag 1440
   cagttcttca gatggaatgg ctgctttcaa gggacggtgt tccttcagaa gctgcagaac 1500
   atttgaaagc tctgataaac agtgtaataa aaataatgag tactgtgaaa aaggtgaaat 1560
   cagagcaact tcatcattcc atgtgcacaa ggaaaagaca ccggcgttgt gagtattccc 1620
   acttc 1625
<210> 9
   <211> 14
   <212> PRT
   <213> Artificial Sequence
<220>
   <223> Nucleoplasmin nuclear localization sequence
<400> 9
   Arg Pro Ala Ala Thr Lys Lys Ala Gly Gln Ala Lys Lys Lys
   1               5                   10